# EUROPEAN PATENT APPLICATION

(11) **EP 4 782 555 A1**
(43) Date of publication of application: **29.07.2026**
(21) Application number: 25154145.4
(22) Date of filing: 27.01.2025
(51) Int. Cl.: C12Q 1/6886, G16B 25/00, G16B 40/00

(54) **METHODS FOR ANALYSING AND PREDICTING RESPONSE TO A IMMUNE CHECKPOINT BLOCKADE THERAPY**

(71) Applicant: Novigenix SA, 1066 Epalinges (CH)
(72) Inventor: CROCI, Davide, 1066 Epalinges (CH); CIARLONI, Laura, 1066 Epalinges (CH); FONSECA, Sara, 1066 Epalinges (CH); HOSSEINIAN EHRENSBERGER, Sahar, 1066 Epalinges (CH); MEHRA, Niven, 6525 GA Nijmegen (NL)
(74) Representative: KATZAROV S.A.

(57) **Abstract**

The present invention relates to methods for predicting or determining if a patient having a cancer will respond, or is responsive, to a treatment based on immune checkpoint blockade therapy or treatment (ICBT). Methods of treatment and kits are also provided.

## Description

### FIELD OF THE INVENTION

The present invention relates to methods for predicting or determining if a patient having a cancer will respond, or is responsive, to a treatment based on immune checkpoint blockade therapy or treatment (ICBT). Methods of treatment and kits are also provided.

### BACKGROUND OF THE INVENTION

During tumor development, cancer cells, together with other cellular and non-cellular components of the tumor microenvironment (TME), orchestrate a plethora of evolutionary adaptations that eventually lead to tumor growth (1,2). A central process enabling cancer cell proliferation is the avoidance of destruction by the immune system (1). Indeed, during every individual's life, the immune system recognizes and eliminates unwanted proliferating cancer cells. This process has been described as the cancer-immunity cycle and includes key immunological steps such as antigen release from cancer cells, T cell priming in the lymph nodes, T cell trafficking to the cancer site, and immunological synapse (3). Tumors have developed several mechanisms to escape such immune surveillance at different stages and sustain their growth. For example, T cell inhibitory signals, such as programmed death-ligand 1 (PD-L1) and its receptor, programmed cell death protein 1 (PD-1), have been shown to pause T cell-mediated cancer cell killing. These signals have been effectively targeted with immune-checkpoint inhibitors (ICIs) to restore the cancer-immunity cycle.

Indeed, ICIs have revolutionized the therapeutic landscape of cancer, providing significant clinical benefit, and even cures in some patients, across a growing number of tumor types. Nine monoclonal antibodies blocking either PD-1 or PD-L1 have been approved by the US Food and Drug Administration (FDA) for the treatment of cancer patients (4). Infusion of these antibodies, alone or in combination, provides enhanced overall response rates (ORR) and improved progression-free survival (PFS) and overall survival (OS) compared to standard treatment or placebo in various tumor types, particularly in patients whose tumors are immunogenic. Of note, response rates to ICIs vary by cancer type and on an individual patient level, and a significant proportion of patients treated with ICIs does not benefit long-term but still must deal with potential immune related adverse events (irAEs). In this context, precise actionable biomarkers predicting immunotherapy response are urgently needed to better guide oncologists in treatment choice and patient stratification, to improve survival and quality of life, and to accelerate and optimize drug development.

Importantly, response-predictive biomarkers include PD-L1 expression in tumor or infiltrating immune cells, tumor mutational burden, and MSI status (5), but show major limitations in precisely anticipating the response to therapy. For instance, PD-L1 tissue expression assessment by immunohistochemistry (IHC) staining has been shown to correlate with therapy response in several cancer types and led to an FDA-approved companion diagnostic test (6). Yet, this approach is limited and cannot be generalized to a global patient population (7). Specifically, PD-L1 IHC staining relies on tumor biopsies, which have the technical disadvantage of being difficult to obtain, especially in longitudinal studies. Moreover, tissue biopsies do not recapitulate the full TME biology and heterogeneity, which is additionally challenging using only a single marker. Other approaches have been developed based on the knowledge that an increased mutational load is associated with a larger variety of cancer neo-antigens (7). Indeed, several biomarkers linked to mutational load have been shown to predict response to ICIs, including tumor mutation burden (8), microsatellite instability (9), and alterations in genes generally involved in DNA damage response, such as BRCA (10). Yet, such biomarkers, although promising, lack complete recapitulation of the TME and the systemic immune response (7,9).

In the last decade, ICIs targeting programmed cell death 1 (PD-1) or its major ligand (PD-L1) have become one of the main treatment modalities for patients with irresectable or metastatic urothelial cancer (mUC). In 2017, pembrolizumab became the standard of care treatment for patients with mUC following progression on first-line platinum-based chemotherapy based on results of the KEYNOTE-045 (11). Since then, the use of ICI in patient with mUC has shifted to the first-line and maintenance setting. In 2021, maintenance therapy with avelumab became available for patients with a response or stable disease to first-line platinum-based chemotherapy (12).

Very recently, the combination of pembrolizumab and antibody-drug conjugate enfortumab-vedotin (EV) became the new standard of care first-line treatment based on results of the EV-302. In this phase III clinical trial, pembrolizumab-EV prolonged median overall survival (mOS) from 16.1 to 31.5 months compared to platinum-based chemotherapy in the first-line setting (13,14).

Another new first-line treatment option for patients who are eligible for cisplatin is the combination of nivolumab with cisplatin and gemcitabine, which has shown an OS advantage compared to cisplatin-based chemotherapy alone in the CheckMate 901 trial (15).

Although ICI-containing combination therapies have proven their superiority compared to first-line chemotherapy in unselected fit patients (13-15), it is anticipated that monotherapy ICI will continue to be an important treatment modality. First, ICI monotherapy will continue to play an important role in the treatment of frail or elderly patients with mUC because of the high toxicity associated with combination therapies. In addition, there might be a role for monotherapy ICI in biomarker-selected patients who are predicted to durably benefit from ICI monotherapy, regardless of frailty, to avert unnecessary toxicity and costs.

Responses to ICI in mUC are heterogeneous. Specifically, monotherapy ICI induces objective response in 20-25% of mUC patients receiving first- or second-line ICI and approximately 10% is still progression-free after 4 years (16,17). These latter patients might not derive extra benefit from the addition of EV or chemotherapy.

To personalize treatment decisions in cancer, and in particular in mUC, there is a need for high precision biomarkers that can identify patients who benefit from ICI monotherapy. Several baseline tumor biomarkers, including tumor mutational burden (TMB), PD-L1 expression and tumor immune cell infiltration, have been associated with response to ICI in mUC (18-22). Although PD-L1 expression enriches for responders to first-line ICI in cisplatin-ineligible patients and is used to select patients for ICI over carboplatin-based chemotherapy, none of these standalone biomarkers are accurate enough to predict response to ICI.

In recent years, circulating tumor DNA (ctDNA) measurement has emerged as a method to monitor treatment response (23-25). The ctDNA level correlates well with tumor burden and can, therefore, be used as a non-invasive tool to monitor treatment response. However, ctDNA does not capture all host-related and tumor microenvironment-related factors that play a role in antitumor immunity.

The current state of immunotherapy response biomarkers highlights an urgent need to develop and implement additional unique, or even composite, predictive biomarkers of clinical benefit, which encompass both tumor and systemic immunity while allowing precise and longitudinal monitoring of patients.

### SUMMARY OF THE INVENTION

The present invention provides a method for predicting or determining if a patient having a cancer will respond, or is responsive, to a treatment based on immune checkpoint blockade therapy or treatment (ICBT), said method comprising determining at one (t1) and/or more time points (t2, t3, ...) in one or more biological samples obtained from said patient having a cancer, the level of transcription and/or expression and/or activity of a gene panel of at least 5 genes, at least 7 genes, at least 10 genes selected from **Table 1,** and wherein differential transcription and/or expression and/or activity level of the gene panel relative to the level of corresponding transcription and/or expression and/or activity level of the gene panel determined previously, is predictive of the patient's response to said treatment.

Also provided is a computer-implemented method for implementing a method for predicting or determining if a patient having a cancer will respond, or is responsive, to a treatment based on immune checkpoint blockade therapy or treatment (ICBT), said computer-implemented method comprising:
i) performing RNA sequencing on a biological sample obtained from said patient having a cancer, to determine at one or more time points in at least one or more biological samples obtained from said patient having a cancer, the level of transcription and/or expression and/or activity of a gene panel of at least 5 genes, at least 7 genes, at least 10 genes selected from **Table 1,**
ii) expressing the differential transcription and/or expression and/or activity level of the gene panel as a gene expression metric,
iii) inputting the gene expression metric into an algorithm that was trained using the same gene expression metric obtained from patients having the cancer, preferentially the same cancer, and treated with a treatment based on ICBT and for which response status (responder (CB) or non-responder (N-CB)) is known,
iv) outputting a probability score.

Also provided is the use of a method of the invention.

Also provided is the use of at least one gene, at least 5 genes, at least 7 genes, at least 10 genes selected from Table 1 in a method for predicting or determining if a patient having a cancer will respond, or is responsive, to a treatment based on ICBT.

Also provide is a gene panel, comprising, or consisting of, RAD51, SCARF1, SMPD3, CLC, CD86, PDXK, PTTG1, TOX, CEBPA and ENSG00000279447.

Also provided is a method of treatment and/or prevention comprising:
performing the method for predicting or determining if a patient having a cancer will respond, or is responsive, to a treatment based on ICBT as disclosed herein; and
i) if the patient having a cancer is determined as responsive (responder, CB) to said treatment based on ICBT, the treatment is continued, or
ii) if the patient having a cancer is determined as not responsive (non-responder, N-CB) to said treatment based on ICBT, the method further comprises a step of adapting the treatment.

Also provided is a kit for performing a method of the invention, said kit comprising a) means and/or reagents for determining the level of transcription and/or expression and/or activity of said gene panel in a biological sample from said patient, and b) instructions for use.

Also provided is a method for predicting and/or determining if a patient having a cancer will respond, or is responsive, to a treatment based on immune checkpoint blockade therapy or treatment (ICBT), said method comprising,
i) determining at one (t1) and/or more time points (t2, t3, ...) the level of transcription and/or expression and/or activity of at least one gene in one or more biological samples obtained from said patient having a cancer,
ii) determining a differential transcription and/or expression and/or activity level of the at least one gene by comparing the transcription and/or expression and/or activity level at one (11) or more time points (t2, t3, ...) versus the transcription and/or expression and/or activity level determined previously,
iii) expressing said differential transcription and/or expression and/or activity level as a gene expression metric,
iv) classifying the samples according to the gene expression metric using an algorithm trained on expression data obtained from samples labelled as responder(s) (CB) and non-responder(s) (N-CB) and, optionally, selecting a gene panel comprising at least one gene responding to treatment based on immune checkpoint blockade therapy or treatment (ICBT),
v) determining at one (11) or more time points (t2, t3, ...) in one or more biological samples obtained from said patient having a cancer, the level and/or amount of circulating tumor DNA (ctDNA),
vi) determining a differential level and/or amount of ctDNAs by comparing the level and/or amount at one (11) or more time points (t2, t3, ...) versus the level and/or amount determined previously, and

wherein differential transcription and/or expression and/or activity level of the genes, or the gene panel, relative to the level of corresponding transcription and/or expression and/or activity level of the genes or gene panel determined previously, and
wherein differential level and/or amount of ctDNAs, in the biological sample, determined at one or more time points (t2, t3, ...) after starting a treatment based on ICBT relative to corresponding level and/or amount of ctDNAs determined previously,
are predictive of the patient's response to said treatment.

### DESCRIPTION OF THE FIGURES

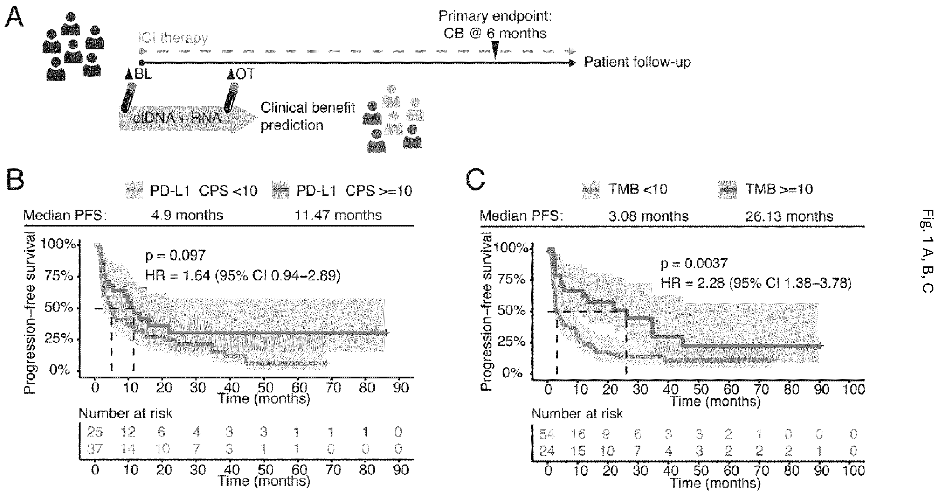

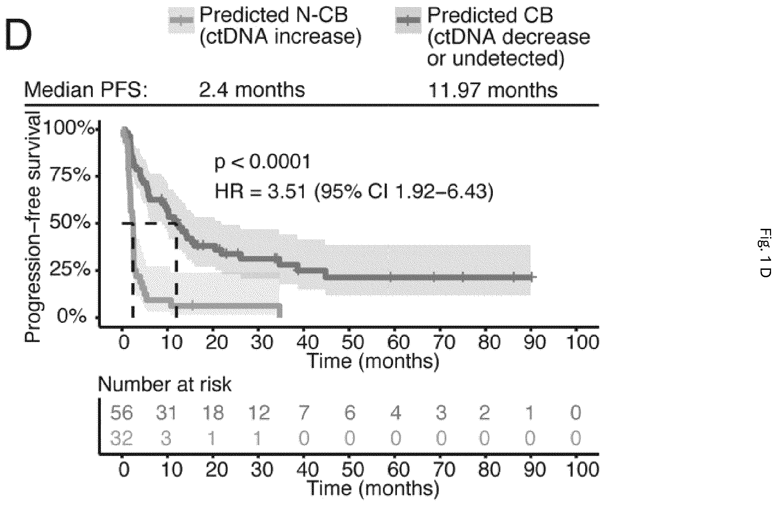

Figure 1- circulating tumor DNA (ctDNA) dynamics predicts clinical benefit (CB) to immune-checkpoint inhibitor (ICI) therapy in metastatic urothelial cancer (mUC) patients: A) Sample collection and analysis schematic: mUC patients were treated with ICI (either pembrolizumab, nivolumab or avelumab) until disease progression. Blood was collected at baseline (BL, before cycle 1) and on-treatment (OT, after 2-6 weeks) for both ctDNA and RNA analysis. The primary endpoint was CB. This was defined as progression-free survival for at least 6 months. B) Kaplan-Meier (KM) curve comparing the progression-free survival (PFS) of patients with PD-L1 positive tumor (dark gray curve, PD-L1 combined positive score >=10) and patients with PD-L1 negative tumor (light gray curve, PD-L1 combined positive score <10). C) KM curve comparing the PFS of patients with a high tumor mutational burden (TMB) (dark gray curve, TMB >=10 mutations/Mb) and TMB low patients (light gray curve, TMB <10). D) KM curve comparing the PFS of ctDNA-based patient stratification. The predicted CB population (dark gray curve) contains patients who had a decrease of ctDNA fraction from BL to OT or undetected at both timepoints. The predicted non-clinical benefit (N-CB, light gray curve) population contains patients where the ctDNA fraction increased from BL to OT or was stable. Statistics: p = p-value as determined by a Mantel-Haenszel test, HR = hazard ratio, CI = confidence interval.

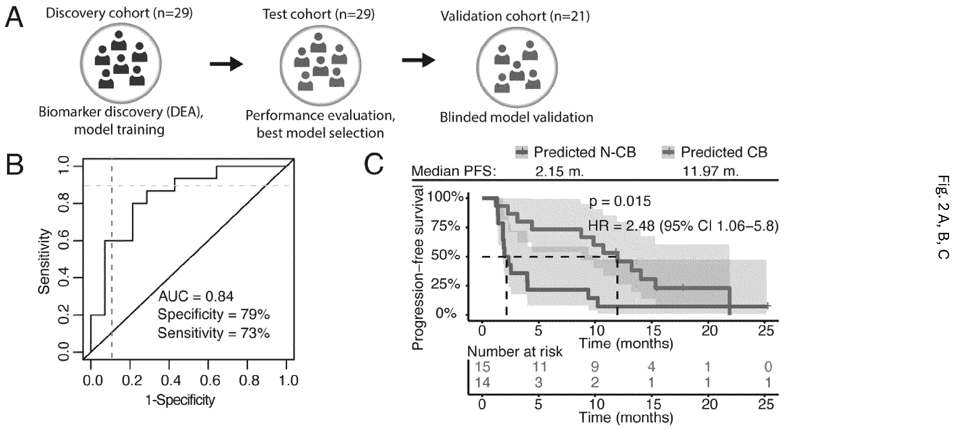

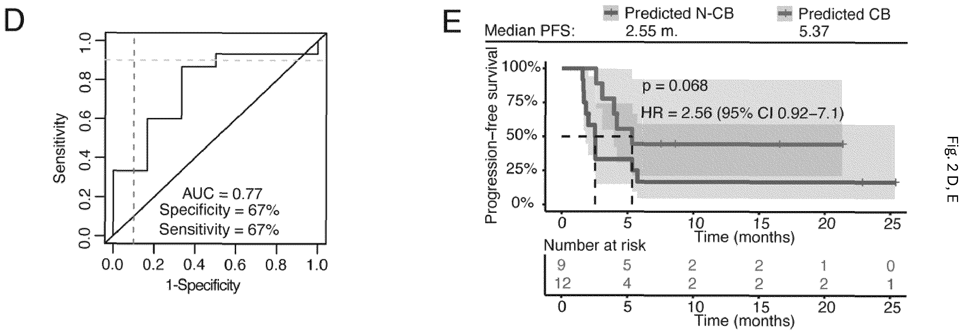

Figure 2 - Blood-based immunotranscriptome predictive model forecasts CB in an independent cohort: A) Modelling approach schematic: biomarker discovery was performed in the discovery cohort (patients with paired BL and OT RNA-seq data, N=29). Model training was performed in the same cohort by multiple iterations of random features reduction of the biomarker/gene list, followed by model testing in the independent test cohort (patients with paired BL and OT RNA-seq data, N=29). The best CB predictive model was selected by area under the curve (AUC) ranking of each model receiver operating characteristics curve (ROC) and by ranking the difference in median PFS between the predicted CB and N-CB groups in the test cohort (N=29). Last, the best performing model was validated in the validation cohort (patients with paired BL and OT RNA-seq data, N=21). B) Receiver-operating characteristics (ROC) curve showing model performance of the best performing model in the independent test cohort (N=29). Specificity is calculated with respect to CB patients (true negative cases), while sensitivity to N-CB (true positive cases). C) KM curve comparing the PFS of model-based predicted CB population and predicted N-CB population in the independent test cohort (N=29). **D)** ROC curve showing model performance assessment in the independent blinded validation cohort (N=21). Specificity is calculated with respect to CB patients (true negative cases) and sensitivity to N-CB (true positive cases). **E)** KM curve comparing the PFS of RNA model-based predicted CB population and N-CB population in the independent blinded validation cohort (N=21). **Statistics:** p = p-value as determined by a Mantel-Haenszel test, HR = hazard ratio (predicted CB population as reference), CI = confidence interval.

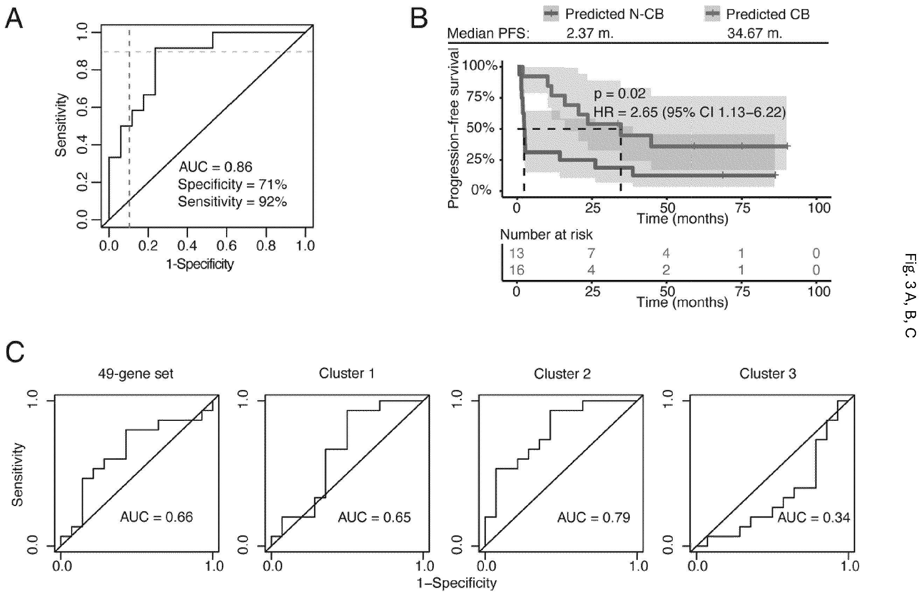

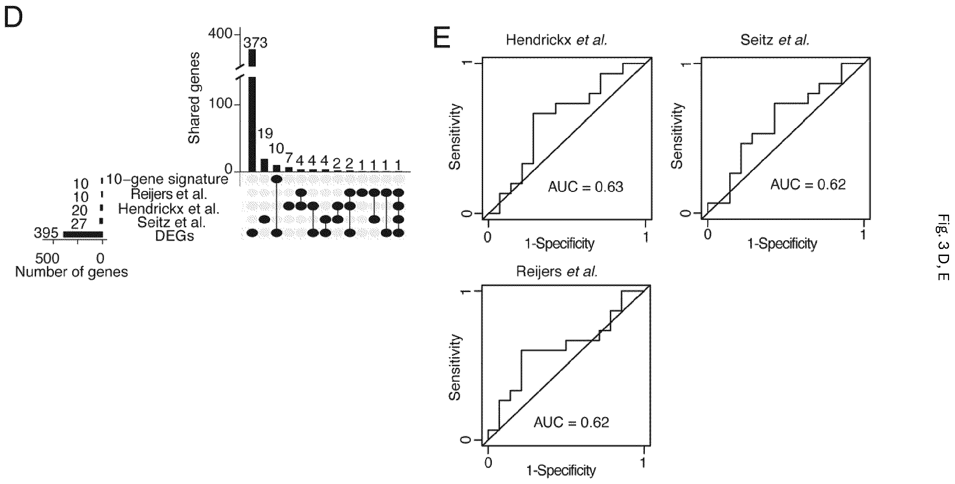

**Figure 3** **- The unique 10-gene panel model outperforms other gene signatures: A)** ROC curve showing model performance in the discovery cohort (N=29). Specificity is calculated with respect to CB patients (true negative cases), while sensitivity to N-CB (true positive cases). **B)** KM curve comparing the PFS of model-based predicted CB population and predicted N-CB population in the discovery cohort (N=29). **C)** ROCs showing the performance in the independent test cohort of CB predictive models crafted with other gene sets from table 1. **D)** Upset plot comparing the identified biomarkers (DEGs) and the 10-gene panel to relevant literature-based tumor-derived gene signatures that have been described to be associated with CB to ICI in other studies (26-28). **E)** ROCs showing the performance in the independent test cohort of CB predictive models crafted with literature-based gene lists. **Statistics:** p = p-value as determined by a Mantel-Haenszel test, HR = hazard ratio (predicted CB population as reference), CI = confidence interval.

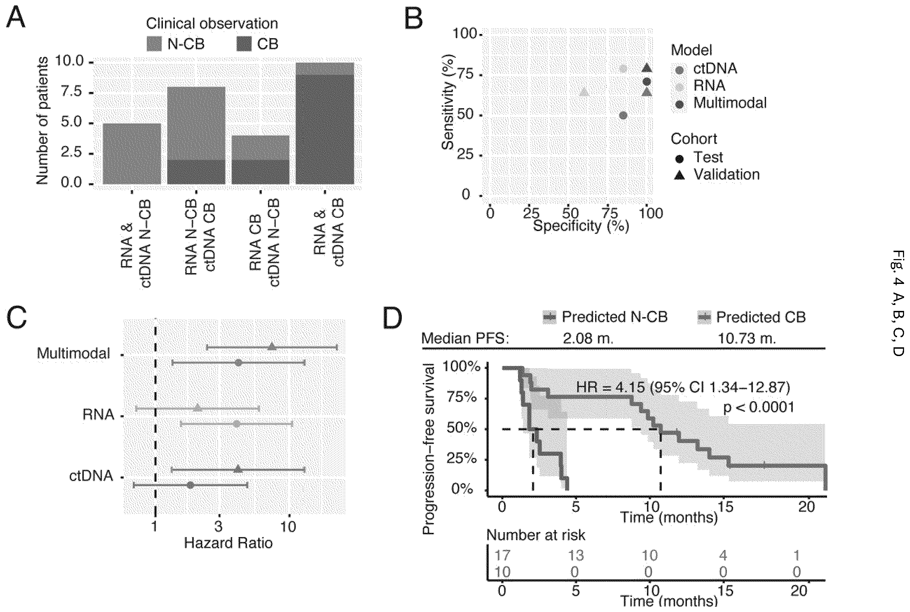

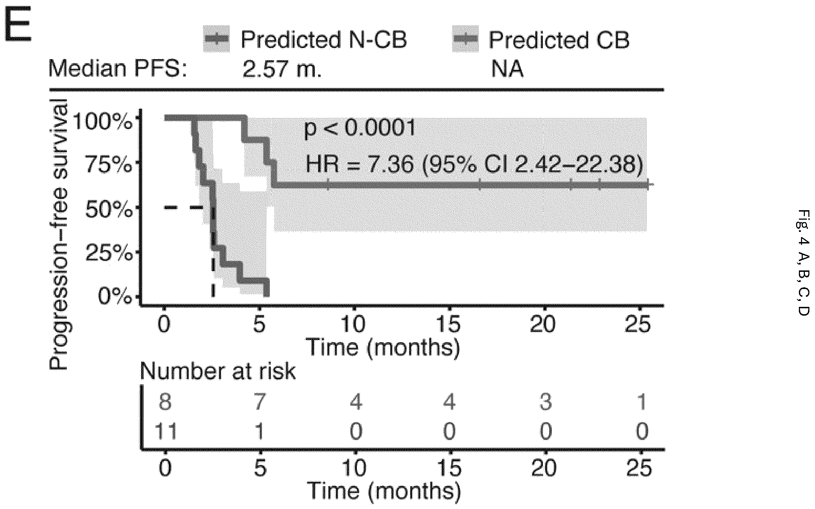

**Figure 4** **- integration of ctDNA- and RNA-based biomarkers boosts the performance of a multimodal model in an independent blinded validation cohort: A)** Prediction comparison: patients of the independent test cohort (N=27, where both RNAseq and ctDNA data were available) were categorized based on the RNA and ctDNA model predictions, highlighting convergent or divergent readouts by the two approaches. Columns' color-coding reflects the actual CB group defined by clinical assessment (dark gray=CB, light gray=N-CB). **B)** Model performance comparison of the different model approaches in the independent test cohort (circles, N=27, where both RNAseq and ctDNA data were available) and blinded validation cohort (triangles, N=19, where both RNAseq and ctDNA data were available). **C)** Hazard Ratio (HR) for PFS of the three modelling approaches used for patient stratification in the independent test (circles, N=27, where both RNAseq and ctDNA data were available) and blinded validation cohorts (triangles, N=19, where both RNAseq and ctDNA data were available). The bars represent the confidence of interval for each HR. The dashed line represents a HR=1. **D)** KM curve comparing the PFS of the multimodal model-based predicted CB population and N-CB population in the independent test cohort (N=27, where both RNAseq and ctDNA data were available) and **E)** in an additional blinded and independent validation cohort (N=19, where both RNAseq and ctDNA data were available). **Statistics:** p = p-value as determined by a Mantel-Haenszel test, HR = hazard ratio (predicted CB population as reference), CI = confidence interval.

### DESCRIPTION OF THE INVENTION

Although methods and materials similar to or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of "include/including", that is to say permitting the presence of one or more features or components. The terms "comprise(s)" and "comprising" also encompass the more restricted ones "consist(s)", "consisting" as well as "consist/consisting essentially of", respectively.

As used in the specification and claims, the singular form "a", "an" and "the" include plural references unless the context clearly dictates otherwise.

As used herein, "at least one" means "one or more", "two or more", "three or more", *etc.* For example, at least one gene means one or more, two or more, three or more, four or more, *etc...*

The term "about", particularly in reference to a given quantity, number or percentage, is meant to encompass deviations of plus or minus ten percent (± 10%). For example, about 5 encompasses any value between 4.5 to 5.5, such as 4.5, 4.6, 4.7, 4.8, 4.9, 5, 5.1, 5.2, 5.3, 5.4, or 5.5.

As used herein the terms "subject"/"patient", are well-recognized in the art, and are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human patient having a cancer.

As used herein, "Cancer" refers to all types of solid or liquid cancer, neoplasm or malignant tumors found in mammals, including leukemias, lymphomas, melanomas, neuroendocrine tumors, carcinomas and sarcomas. Exemplary cancers may be is selected from the non-limiting group comprising urothelial cancer, urinary bladder cancer (e.g. muscle-invasive bladder cancer (MIBC)), lung cancer, breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, hepatic cancer, brain cancer and skin cancer (e.g. melanoma), or a combination of two or more thereof.

"Leukemia" refers broadly to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease-acute or chronic; (2) the type of cell involved; myeloid (myelogenous), lymphoid (lymphogenous), or monocytic; and (3) the increase or non-increase in the number abnormal cells in the blood-leukemic or aleukemic (subleukemic). Exemplary leukemias that may be treated with a compound, pharmaceutical composition, or method provided herein include, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocyte leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, multiple myeloma, plasmacytic leukemia, promyelocytic leukemia, Rieder cell leukemia, Schilling's leukemia, stem cell leukemia, subleukemic leukemia, or undifferentiated cell leukemia.

"Sarcoma" generally refers to a tumor which is made up of a substance like the embryonic connective tissue and is generally composed of closely packed cells embedded in a fibrillar or homogeneous substance. Sarcomas that may be treated with a compound, pharmaceutical composition, or method provided herein include a chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, or telangiectaltic sarcoma.

"Melanoma" is taken to mean a tumor arising from the melanocytic system of the skin and other organs. Melanomas that may be treated with a compound, pharmaceutical composition, or method provided herein include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, or superficial spreading melanoma.

"Carcinoma" refers to a malignant new growth made up of epithelial cells tending to infiltrate the surrounding tissues and give rise to metastases. Exemplary carcinomas that may be treated with a compound, pharmaceutical composition, or method provided herein include, for example, medullary thyroid carcinoma, familial medullary thyroid carcinoma, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basal oid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, ductal carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniforni carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lobular carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tubular carcinoma, tuberous carcinoma, verrucous carcinoma, or carcinoma villosum.

"Metastasis," "metastatic," and "metastatic cancer" can be used interchangeably and refer to the spread of a proliferative disease or disorder, e.g., cancer, from one organ or another non-adjacent organ or body part. Cancer occurs at an originating site, e.g., breast, which site is referred to as a primary tumor, e.g., primary breast cancer. Some cancer cells in the primary tumor or originating site acquire the ability to penetrate and infiltrate surrounding normal tissue in the local area and/or the ability to penetrate the walls of the lymphatic system or vascular system circulating through the system to other sites and tissues in the body. A second clinically detectable tumor formed from cancer cells of a primary tumor is referred to as a metastatic or secondary tumor. When cancer cells metastasize, the metastatic tumor and its cells are presumed to be similar to those of the original tumor. Thus, if lung cancer metastasizes to the breast, the secondary tumor at the site of the breast consists of abnormal lung cells and not abnormal breast cells. The secondary tumor in the breast is referred to a metastatic lung cancer. Thus, the phrase metastatic cancer refers to a disease in which a subject has or had a primary tumor and has one or more secondary tumors. The phrases non-metastatic cancer or subjects with cancer that is not metastatic refers to diseases in which subjects have a primary tumor but not one or more secondary tumors. For example, metastatic lung cancer refers to a disease in a subject with or with a history of a primary lung tumor and with one or more secondary tumors at a second location or multiple locations, e.g., in the breast.

The terms "sample" or "biological sample" as used herein include any biological specimen obtained from a subject or patient. Biological samples include a body fluid, a body cell or a tissue. Biological samples may include, without limitation, whole blood, plasma, serum, red blood cells, white blood cells (e.g., peripheral blood mononuclear cells), saliva, urine, stool (i.e., fecal material), tears, sweat, buccal smears, skin, sebum, nipple aspirate, ductal lavage, tumour exudates, tissue such as tumor tissue, cancer cells, synovial fluid, cerebrospinal fluid, lymph, fine needle aspirate, amniotic fluid, any other bodily fluid, cell lysates, cellular secretion products, inflammation fluid, semen and vaginal secretions, or a combination of one or more of thereof. In some aspects, the biological sample also includes extracellular vesicles such as, e.g. exosomes and microvesicles that are two major categories of extracellular vesicles released by almost all cell types and are highly abundant in biological fluids.

In preferred aspects, the sample may be whole blood or a fractional component thereof such as, e.g., plasma, serum, a cell pellet or a tumor tissue (e.g. a biopsy). According to a specific aspect, the sample used in the method according to the invention is whole blood or a fractional component thereof, such as e.g. white blood cells or peripheral blood mononuclear cells (PBMC) or tumor tissue.

Preferably the sample is readily obtainable by minimally invasive methods.

As used herein, "ICBT" and "ICI" are used interchangeably herein to refer to immunotherapy treatment based on immune checkpoint blockade therapy or treatment.

In one aspect, the treatment of the invention is an immunotherapy treatment based on immune checkpoint blockade therapy or treatment (ICBT). Preferably, said treatment based on ICBT is selected among the group comprising a PD-1 inhibitor, a PD-L1 inhibitor, a LAG-3 inhibitor, a TIM-3 inhibitor, a TIGIT inhibitor, a BTLA inhibitor and a CTLA-4 inhibitor, or combination of one or more thereof (e.g. CTLA-4 and PD-1 inhibitors, or LAG-3 and PD-1 inhibitors).

In a preferred aspect, the treatment based on ICBT comprises treatment with monoclonal antibodies (mAbs) specific to, or designed to bind with, PD-1, PD-L1, LAG-3, TIM-3, TIGIT, BTLA or CTLA-4, or a combination of one or more thereof (see e.g. Rotte, A. et al., 2019; Twomey, J.D. and Zhang, B. 2021).

Non-limiting examples of mAbs specific to PD-1 comprise nivolumab, pembrolizumab, dostarlimab, retifanlimab and cemiplimab.

Non- limiting examples of mAbs specific to PDL-1 comprise atezolizumab, avelumab, and durvalumab.

Non- limiting examples of mAbs specific to LAG-3 comprise favezelimab and relatlimab. Non- limiting examples of mAbs specific to TIM-3 comprise cobolimab, LY3321367, or sabatolimab.

Non- limiting examples of mAbs specific to TIGIT comprise tiragolumab, EOS-448 (developed by GSK and iTeos Therapeutics) and Domvanalimab (Gilead/Arcus).

Non- limiting examples of mAbs specific to BTLA comprise IND (junishi biosciences) and talquetamab-tgvs.

Non- limiting examples of mAb specific to CTLA-4 consist of ipilimumab and tremelimumab.

In one aspect, the treatment based on ICBT further comprises treatment with an additional therapy selected from chemotherapy, immunotherapy, targeted therapy (such as e.g. Tyrosine Kinase Inhibitor, Antibody Drug Conjugates) or radiotherapy or combination of one or more thereof.

As discussed herein, a "score" may be calculated as the mean, or the median, or the ratio or the sum, or the weighted mean, median or the sum, the ratio of the level and/or amount of ctDNAs in control samples (e.g. reference samples, baseline, ...) or level of transcription and/or expression and/or activity of at least one gene of the invention and disease samples. In some spasects, the score is an expression metric.

The present invention provides a method for predicting or determining if a patient having a cancer will respond, or is responsive, to a treatment based on immune checkpoint blockade therapy or treatment (ICBT), said method comprising determining at one (t1) and/or more time points (t2, t3, ...) in one or more biological samples obtained from said patient having a cancer, the level of transcription and/or expression and/or activity of at least one gene selected from **Table 1,** and wherein differential transcription and/or expression and/or activity level of the at least gene relative to the level of corresponding transcription and/or expression and/or activity level of the at least one gene determined previously, is predictive of the patient's response to said treatment.

Preferably, the at least one gene selected from **Table 1** comprises a gene panel (i.e. a combination) of at least two genes, at least 5 genes, at least 7 genes, at least 10 genes selected from **Table 1.**

According to one aspect of the invention, the gene panel selected from **Table 1** comprises RAD51, SCARF 1 (scavenger receptor class F member), SMPD3 (Sphingomyelin Phosphodiesterase 3), CLC (Charcot-Leyden Crystal Galectin), CD86 (Cluster of Differentiation 86), PDXK (Pyridoxal Kinase), PTTG1 (Pituitary tumor-transforming gene 1), TOX (thymocyte selection associated high mobility group box), CEBPA ((CCAAT Enhancer Binding Protein Alpha) and ENSG00000279447 (Ensembl version 107).

According to one aspect of the invention, the gene panel selected from **Table 1** consists of RAD51, SCARF1, SMPD3, CLC, CD86, PDXK, PTTG1, TOX, CEBPA and ENSG00000279447.

### Table 1

**Table 1: list of genes. Ensembl ID based on Ensembl version 107.**

| **ensembl ID** | **symbol** |
|---|---|
| ENSG00000051180 | RAD51 |
| ENSG00000074660 | SCARF1 |
| ENSG00000103056 | SMPD3 |
| ENSG00000105205 | CLC |
| ENSG00000114013 | CD86 |
| ENSG00000160209 | PDXK |
| ENSG00000164611 | PTTG1 |
| ENSG00000198846 | TOX |
| ENSG00000279447 | ENSG00000279447 |
| ENSG00000245848 | CEBPA |
| ENSG00000002549 | LAP3 |
| ENSG00000004399 | PLXND1 |
| ENSG00000006282 | SPATA20 |
| ENSG00000006530 | AGK |
| ENSG00000008283 | CYB561 |
| ENSG00000010030 | ETV7 |
| ENSG00000010278 | CD9 |
| ENSG00000010318 | PHF7 |
| ENSG00000010327 | STAB1 |
| ENSG00000026103 | FAS |
| ENSG00000026950 | BTN3A1 |
| ENSG00000028277 | POU2F2 |
| ENSG00000038427 | VCAN |
| ENSG00000043514 | TRIT1 |
| ENSG00000047365 | ARAP2 |
| ENSG00000048140 | TSPAN17 |
| ENSG00000051128 | HOMER3 |
| ENSG00000057657 | PRDM1 |
| ENSG00000064886 | CH13L2 |
| ENSG00000065054 | SLC9A3R2 |
| ENSG00000065485 | PDIA5 |
| ENSG00000065491 | TBC1D22B |
| ENSG00000067221 | STOML1 |
| ENSG00000069509 | FUNDC1 |
| ENSG00000070501 | POLB |
| ENSG00000071205 | ARHGAP10 |
| ENSG00000071246 | VASH1 |
| ENSG00000072134 | EPN2 |
| ENSG00000073008 | PVR |
| ENSG00000073060 | SCARB1 |
| ENSG00000076003 | MCM6 |
| ENSG00000077684 | JADE1 |
| ENSG00000082515 | MRPL22 |
| ENSG00000083838 | ZNF446 |
| ENSG00000084207 | GSTP1 |
| ENSG00000087253 | LPCAT2 |
| ENSG00000088325 | TPX2 |
| ENSG00000089169 | RPH3A |
| ENSG00000089327 | FXYD5 |
| ENSG00000089692 | LAG3 |
| ENSG00000091181 | IL5RA |
| ENSG00000092010 | PSME1 |
| ENSG00000092067 | CEBPE |
| ENSG00000092068 | SLC7A8 |
| ENSG00000092847 | AGO1 |
| ENSG00000095585 | BLNK |
| ENSG00000096996 | IL12RB1 |
| ENSG00000097046 | CDC7 |
| ENSG00000099330 | OCEL1 |
| ENSG00000099958 | DERL3 |
| ENSG00000100079 | LGALS2 |
| ENSG00000100162 | CENPM |
| ENSG00000100211 | CBY1 |
| ENSG00000100336 | APOL4 |
| ENSG00000100342 | APOL1 |
| ENSG00000100450 | GZMH |
| ENSG00000100519 | PSMC6 |
| ENSG00000100628 | ASB2 |
| ENSG00000100911 | PSME2 |
| ENSG00000101439 | CST3 |
| ENSG00000102145 | GATA1 |
| ENSG00000102181 | CD99L2 |
| ENSG00000102445 | RUBCNL |
| ENSG00000102575 | ACP5 |
| ENSG00000102878 | HSF4 |
| ENSG00000103355 | PRSS33 |
| ENSG00000104081 | BMF |
| ENSG00000104518 | GSDMD |
| ENSG00000104738 | MCM4 |
| ENSG00000105255 | FSD1 |
| ENSG00000105366 | SIGLEC8 |
| ENSG00000105374 | NKG7 |
| ENSG00000105383 | CD33 |
| ENSG00000105662 | CRTC1 |
| ENSG00000106123 | EPHB6 |
| ENSG00000106462 | EZH2 |
| ENSG00000106560 | GIMAP2 |
| ENSG00000106991 | ENG |
| ENSG00000107020 | PLGRKT |
| ENSG00000107551 | RASSF4 |
| ENSG00000107960 | STN1 |
| ENSG00000108588 | CCDC47 |
| ENSG00000108784 | NAGLU |
| ENSG00000109065 | NAT9 |
| ENSG00000109805 | NCAPG |
| ENSG00000109861 | CTSC |
| ENSG00000111110 | PPM1H |
| ENSG00000111252 | SH2B3 |
| ENSG00000111275 | ALDH2 |
| ENSG00000111801 | BTN3A3 |
| ENSG00000112137 | PHACTR1 |
| ENSG00000112312 | GMNN |
| ENSG00000112695 | COX7A2 |
| ENSG00000112759 | SLC29A1 |
| ENSG00000112799 | LY86 |
| ENSG00000113088 | GZMK |
| ENSG00000114127 | XRN1 |
| ENSG00000114302 | PRKAR2A |
| ENSG00000114423 | CBLB |
| ENSG00000114648 | KLHL18 |
| ENSG00000114770 | ABCC5 |
| ENSG00000115415 | STAT1 |
| ENSG00000115446 | UNC50 |
| ENSG00000115602 | IL1RL1 |
| ENSG00000115738 | ID2 |
| ENSG00000116663 | FBXO6 |
| ENSG00000116729 | WLS |
| ENSG00000116990 | MYCL |
| ENSG00000117226 | GBP3 |
| ENSG00000117228 | GBP1 |
| ENSG00000117399 | CDC20 |
| ENSG00000117560 | FASLG |
| ENSG00000117632 | STMN1 |
| ENSG00000119866 | BCL11A |
| ENSG00000119912 | IDE |
| ENSG00000119929 | CUTC |
| ENSG00000120708 | TGFBI |
| ENSG00000120875 | DUSP4 |
| ENSG00000120896 | SORBS3 |
| ENSG00000120949 | TNFRSF8 |
| ENSG00000121691 | CAT |
| ENSG00000123384 | LRP1 |
| ENSG00000124191 | TOX2 |
| ENSG00000124225 | PMEPA1 |
| ENSG00000125733 | TRIP10 |
| ENSG00000125743 | SNRPD2 |
| ENSG00000127540 | UQCR11 |
| ENSG00000127564 | PKMYT1 |
| ENSG00000128284 | APOL3 |
| ENSG00000128973 | CLN6 |
| ENSG00000129667 | RHBDF2 |
| ENSG00000130222 | GADD45G |
| ENSG00000130304 | SLC27A1 |
| ENSG00000130433 | CACNG6 |
| ENSG00000131203 | IDO1 |
| ENSG00000131979 | GCH1 |
| ENSG00000132274 | TRIM22 |
| ENSG00000132535 | DLG4 |
| ENSG00000133106 | EPSTI1 |
| ENSG00000133321 | PLAAT4 |
| ENSG00000134056 | MRPS36 |
| ENSG00000134690 | CDCA8 |
| ENSG00000134955 | SLC37A2 |
| ENSG00000135093 | USP30 |
| ENSG00000135362 | PRR5L |
| ENSG00000135912 | TTLL4 |
| ENSG00000135916 | ITM2C |
| ENSG00000136059 | VILL |
| ENSG00000136514 | RTP4 |
| ENSG00000136770 | DNAJC1 |
| ENSG00000136888 | ATP6V1G1 |
| ENSG00000137413 | TAF8 |
| ENSG00000137441 | FGFBP2 |
| ENSG00000137726 | FXYD6 |
| ENSG00000137880 | GCHFR |
| ENSG00000138160 | KIF11 |
| ENSG00000138623 | SEMA7A |
| ENSG00000138744 | NAAA |
| ENSG00000139180 | NDUFA9 |
| ENSG00000140090 | SLC24A4 |
| ENSG00000140263 | SORD |
| ENSG00000140450 | ARRDC4 |
| ENSG00000140525 | FANCI |
| ENSG00000140836 | ZFHX3 |
| ENSG00000141504 | SAT2 |
| ENSG00000141505 | ASGR1 |
| ENSG00000141540 | TTYH2 |
| ENSG00000141753 | IGFBP4 |
| ENSG00000143476 | DTL |
| ENSG00000143819 | EPHX1 |
| ENSG00000144354 | CDCA7 |
| ENSG00000145365 | TIFA |
| ENSG00000145649 | GZMA |
| ENSG00000145685 | LHFPL2 |
| ENSG00000145990 | GFOD1 |
| ENSG00000146540 | C7orf50 |
| ENSG00000147036 | LANCL3 |
| ENSG00000147155 | EBP |
| ENSG00000147889 | CDKN2A |
| ENSG00000148344 | PTGES |
| ENSG00000148773 | MKI67 |
| ENSG00000149639 | SOGA1 |
| ENSG00000149809 | TM7SF2 |
| ENSG00000150337 | FCGR1A |
| ENSG00000151014 | NOCT |
| ENSG00000151715 | TMEM45B |
| ENSG00000151914 | DST |
| ENSG00000152082 | MZT2B |
| ENSG00000152229 | PSTPIP2 |
| ENSG00000152242 | C18orf25 |
| ENSG00000152969 | JAKMIP1 |
| ENSG00000153563 | CD8A |
| ENSG00000153574 | RPIA |
| ENSG00000154451 | GBP5 |
| ENSG00000154582 | ELOC |
| ENSG00000154723 | ATP5PF |
| ENSG00000156127 | BATF |
| ENSG00000156587 | UBE2L6 |
| ENSG00000156869 | FRRS1 |
| ENSG00000157045 | NTAN1 |
| ENSG00000157168 | NRG1 |
| ENSG00000157227 | MMP14 |
| ENSG00000157456 | CCNB2 |
| ENSG00000157823 | AP3S2 |
| ENSG00000158006 | PAFAH2 |
| ENSG00000158062 | UBXN11 |
| ENSG00000158714 | SLAMF8 |
| ENSG00000159363 | ATP13A2 |
| ENSG00000159388 | BTG2 |
| ENSG00000159618 | ADGRG5 |
| ENSG00000159714 | ZDHHC1 |
| ENSG00000160233 | LRRC3 |
| ENSG00000160293 | VAV2 |
| ENSG00000160791 | CCR5 |
| ENSG00000160957 | RECQL4 |
| ENSG00000161642 | ZNF385A |
| ENSG00000161905 | ALOX15 |
| ENSG00000162390 | ACOT11 |
| ENSG00000162496 | DHRS3 |
| ENSG00000162645 | GBP2 |
| ENSG00000162654 | GBP4 |
| ENSG00000162711 | NLRP3 |
| ENSG00000162739 | SLAMF6 |
| ENSG00000162772 | ATF3 |
| ENSG00000163214 | DHX57 |
| ENSG00000163568 | AIM2 |
| ENSG00000163832 | ELP6 |
| ENSG00000163918 | RFC4 |
| ENSG00000164308 | ERAP2 |
| ENSG00000164530 | P116 |
| ENSG00000164687 | FABP5 |
| ENSG00000165801 | ARHGEF40 |
| ENSG00000165806 | CASP7 |
| ENSG00000166033 | HTRA1 |
| ENSG00000166401 | SERPINB8 |
| ENSG00000166508 | MCM7 |
| ENSG00000166689 | PLEKHA7 |
| ENSG00000166839 | ANKDD1A |
| ENSG00000166928 | MS4A14 |
| ENSG00000168004 | PLAAT5 |
| ENSG00000168209 | DDIT4 |
| ENSG00000168394 | TAP1 |
| ENSG00000168765 | GSTM4 |
| ENSG00000168826 | ZBTB49 |
| ENSG00000168899 | VAMP5 |
| ENSG00000169313 | P2RY12 |
| ENSG00000169583 | CLIC3 |
| ENSG00000169715 | MT1E |
| ENSG00000169957 | ZNF768 |
| ENSG00000170476 | MZB1 |
| ENSG00000171604 | CXXC5 |
| ENSG00000171984 | SHLD1 |
| ENSG00000172057 | ORMDL3 |
| ENSG00000172116 | CD8B |
| ENSG00000172164 | SNTB1 |
| ENSG00000172215 | CXCR6 |
| ENSG00000172819 | RARG |
| ENSG00000172954 | LCLAT1 |
| ENSG00000173193 | PARP14 |
| ENSG00000173221 | GLRX |
| ENSG00000174516 | PEL13 |
| ENSG00000174944 | P2RY14 |
| ENSG00000175866 | BAIAP2 |
| ENSG00000177239 | MAN1B1 |
| ENSG00000177409 | SAMD9L |
| ENSG00000178409 | BEND3 |
| ENSG00000178585 | CTNNBIP1 |
| ENSG00000178999 | AURKB |
| ENSG00000179021 | C3orf38 |
| ENSG00000179044 | EXOC3L1 |
| ENSG00000179958 | DCTPP1 |
| ENSG00000181036 | FCRL6 |
| ENSG00000181523 | SGSH |
| ENSG00000182240 | BACE2 |
| ENSG00000182557 | SPNS3 |
| ENSG00000182578 | CSF1R |
| ENSG00000182580 | EPHB3 |
| ENSG00000183347 | GBP6 |
| ENSG00000183625 | CCR3 |
| ENSG00000184451 | CCR10 |
| ENSG00000185164 | NOMO2 |
| ENSG00000185880 | TRI M69 |
| ENSG00000186047 | DLEU7 |
| ENSG00000186318 | BACE1 |
| ENSG00000186810 | CXCR3 |
| ENSG00000186815 | TPCN1 |
| ENSG00000187741 | FANCA |
| ENSG00000188389 | PDCD1 |
| ENSG00000188820 | CALHM6 |
| ENSG00000188822 | CNR2 |
| ENSG00000189068 | VSTM1 |
| ENSG00000196411 | EPHB4 |
| ENSG00000196576 | PLXNB2 |
| ENSG00000197057 | DTHD1 |
| ENSG00000197142 | ACSL5 |
| ENSG00000197536 | IRF1-AS1 |
| ENSG00000197586 | ENTPD6 |
| ENSG00000198019 | FCGR1BP |
| ENSG00000198826 | ARHGAP11A |
| ENSG00000198846 | TOX |
| ENSG00000198853 | RUSC2 |
| ENSG00000204054 | LINC00963 |
| ENSG00000204161 | TMEM273 |
| ENSG00000204264 | PSMB8 |
| ENSG00000204267 | TAP2 |
| ENSG00000204323 | SMIM5 |
| ENSG00000204406 | MBD5 |
| ENSG00000204642 | HLA-F |
| ENSG00000205220 | PSMB10 |
| ENSG00000205336 | ADGRG1 |
| ENSG00000206337 | HCP5 |
| ENSG00000213337 | ANKRD39 |
| ENSG00000213876 | RPL7AP64 |
| ENSG00000218891 | ZNF579 |
| ENSG00000221963 | APOL6 |
| ENSG00000224505 | HEXIM2-AS1 |
| ENSG00000224616 | RTCA-AS1 |
| ENSG00000225151 | GOLGA2P7 |
| ENSG00000225697 | SLC26A6 |
| ENSG00000226137 | BAIAP2-DT |
| ENSG00000230138 | LINC02812 |
| ENSG00000231113 | LINC02976 |
| ENSG00000231535 | LINC00278 |
| ENSG00000231680 | LINC02723 |
| ENSG00000233016 | SNHG7 |
| ENSG00000234518 | PTGES3P1 |
| ENSG00000235217 | TSPY26P |
| ENSG00000237649 | KIFC1 |
| ENSG00000238121 | LINC00426 |
| ENSG00000239264 | TXNDC5 |
| ENSG00000239713 | APOBEC3G |
| ENSG00000239839 | DEFA3 |
| ENSG00000240065 | PSMB9 |
| ENSG00000240505 | TNFRSF13B |
| ENSG00000243056 | EIF4EBP3 |
| ENSG00000243064 | ABCC13 |
| ENSG00000243811 | APOBEC3D |
| ENSG00000245025 | ENSG00000245025 |
| ENSG00000247315 | ZCCHC3 |
| ENSG00000250264 | ENSG00000250264 |
| ENSG00000250510 | GPR162 |
| ENSG00000250696 | ENSG00000250696 |
| ENSG00000250903 | GMDS-DT |
| ENSG00000251301 | LINC02384 |
| ENSG00000254709 | IGLL5 |
| ENSG00000254838 | GVINP1 |
| ENSG00000255587 | RAB44 |
| ENSG00000256039 | LINC02446 |
| ENSG00000256262 | USP30-AS1 |
| ENSG00000258429 | PDF |
| ENSG00000258818 | RNASE4 |
| ENSG00000260007 | ENSG00000260007 |
| ENSG00000267727 | ENSG00000267727 |
| ENSG00000269404 | SPIB |
| ENSG00000269711 | ENSG00000269711 |
| ENSG00000269713 | NBPF9 |
| ENSG00000269973 | ENSG00000269973 |
| ENSG00000269983 | ENSG00000269983 |
| ENSG00000270800 | RPS10-NUDT3 |
| ENSG00000270882 | H4C14 |
| ENSG00000272625 | ENSG00000272625 |
| ENSG00000272918 | ENSG00000272918 |
| ENSG00000273691 | ENSG00000273691 |
| ENSG00000274020 | LINC01138 |
| ENSG00000274265 | ENSG00000274265 |
| ENSG00000274944 | ENSG00000274944 |
| ENSG00000275302 | CCL4 |
| ENSG00000276043 | UHRF1 |
| ENSG00000276070 | CCL4L2 |
| ENSG00000276278 | ENSG00000276278 |
| ENSG00000277147 | LINC00869 |
| ENSG00000279117 | ENSG00000279117 |
| ENSG00000280106 | ENSG00000280106 |
| ENSG00000280734 | LINC01232 |
| ENSG00000282608 | ADORA3 |
| ENSG00000282851 | BISPR |
| ENSG00000284649 | ENSG00000284649 |
| ENSG00000284820 | ENSG00000284820 |
| ENSG00000284906 | ENSG00000284906 |
| ENSG00000287839 | ENSG00000287839 |
| ENSG00000288587 | ENSG00000288587 |
| ENSG00000288612 | ENSG00000288612 |
| ENSG00000288990 | ENSG00000288990 |
| ENSG00000289561 | ENSG00000289561 |
| ENSG00000289977 | ENSG00000289977 |
| ENSG00000290017 | ENSG00000290017 |

Typically, the level of transcription and/or expression and/or activity of the gene panel is determined at one (t1) and/or more time points (t2, t3, ...) in the biological sample. The determination of the level of transcription and/or expression and/or activity of the gene panel is usually performed after the treatment based on ICBT has started, preferably between about 1 to about 16 weeks, about 2 to about 14 weeks, about 2 to about 12 weeks, about 2 to about 10 weeks, typically at time points t2, t3 . In one aspect, t1 corresponds to a time point before the treatment based on ICBT has started and can serve to determine a baseline (BL).

It is generally understood that t1 precedes t2, that t2 precedes t3, etc...

In another aspect, the determination of the level of transcription and/or expression and/or activity of the gene panel is performed before the treatment based on ICBT has started, preferably between about 1 to about 5 hours, about 1 to about 16 weeks, about 2 to about 14 weeks, about 2 to about 12 weeks, about 2 to about 10 weeks.

According to an aspect of the invention, the level of transcription and/or expression and/or activity of a gene panel may be expressed as a gene expression metric.

The gene expression metric may be any expression level or value, or may be calculated as the mean, or the median, or the ratio or the sum, or the weighted mean, median or the sum, the ratio of the expression levels of the genes composing the panel in control samples (e.g. reference samples, baseline, ...) and disease samples.

Alternatively, the gene expression metric may be calculated as the first component or multiple components of Principal Component Analysis (PCA), or Neural Network dimensional embeddings or any Dimensionality reduction method.

According to an aspect of the invention, the gene expression metric is inputted into an algorithm that was trained using gene expression metric from patients having cancer, preferably the same cancer, and treated with a treatment based on ICBT and for which response status (patient with clinical benefit (CB) or patient without clinical benefit (N-CB)) is known. The trained algorithm further outputs a score, preferentially a Probability Score, usually comprised between (and included) 0 and 1.

In one aspect, the algorithm outputs a probability of a prediction model using generalized linear models (aslogistic regression and svc), or gradient boosting classifier, or decision tree classifier, or random forest classifier, or ada boost classifier, or Lasso and Elastic-Net Regularized Generalized Linear Models, Sparse partial least squares regression, or nearest-centroid classification, or nearest shrunken centroid, or neural networks or or support vector machine, or naive bayes, or K-means.

Various techniques for determining differential transcription and/or expression and/or activity of e.g. a gene or a gene panel are known in the art.

For example, one may calculate differential expression of one gene in a test sample by, e.g. calculating the ratio (or fold change) between the expression level of the gene in the test sample and the expression level of the gene in the reference sample or group of samples, or reference value. In one aspect, the reference sample, group of samples, or reference value has been determined previously or is known and may be from an equivalent biological sample.

Expression level can be measured as transcripts (e.g. mRNAs) per million (TPM) by RNA seq, as Threshold cycles (Ct) by PCR, as probe fluorescence intensity by microarray, *etc....*

The terms "quantity," "amount," and "level" are used interchangeably herein and may refer to an absolute quantification of a molecule or an analyte in a sample, or to a relative quantification of a molecule or analyte in a sample, i.e., relative to another value such as relative to a reference value as taught herein, or to a range of values for the biomarker in the absence of treatment or after starting the treatment. These values or ranges can be obtained from a single patient or alternatively from a group of patients.

The transcripts of the genes of the invention can be detected and, alternatively, quantitated by a variety of methods including, but not limited to, microarray analysis, polymerase chain reaction (PCR), reverse transcriptase polymerase chain reaction (RT-PCR), digital PCR, Northern blot, serial analysis of gene expression (SAGE), immunoassay, mass spectrometry, and any RNA sequencing-based methods known in the art (such as e.g. whole transcriptome RNA seq, targeted RNA seq, single cell RNA seq, total RNA sequencing, mRNA sequencing, whole transcript RNA sequencing, 3' RNA sequencing, long RNA sequencing, direct RNA sequencing), polyA RNA, or to specific target gene transcripts (e.g. targeted RNA seq).

It is understood that the expression level of the genes (e.g. transcripts) in a sample can be determined by any suitable method known in the art. Measurement of the level of a gene can be direct or indirect. For example, the abundance levels of RNAs can be directly quantitated. Alternatively, the amount of a gene can be determined indirectly by measuring abundance levels of cDNAs, amplified RNAs or DNAs, or by measuring quantities or activities of RNAs, or other molecules that are indicative of the expression level of the gene (such as, e.g proteins). Preferably, the amount of a gene is determined indirectly by measuring abundance levels of cDNAs.

Transcripts which may be measured by microarray or RNA sequencing analysis can be expressed RNAs or a nucleic acid derived therefrom (e.g., cDNA or amplified RNA derived from cDNA that incorporates an RNA polymerase promoter), including naturally occurring nucleic acid molecules, as well as synthetic nucleic acid molecules. In one aspect, the target polynucleotide molecules comprise RNA, including, but by no means limited to, total cellular RNA, poly(A)+ messenger RNA (mRNA) or a fraction thereof, cytoplasmic mRNA, or RNA transcribed from cDNA (i.e., cRNA; see, e.g., U.S. Pat. No. 5,545,522, 5,891,636, or 5,716,785). Methods for preparing total and poly(A)+ RNA are well known in the art, and are described generally, e.g., in Sambrook, et al., Molecular Cloning: A Laboratory Manual (3rd Edition, 2001) as well as e.g. in Hong, M., Tao, S., Zhang, L. et al. RNA sequencing: new technologies and applications in cancer research. J Hematol Oncol 13, 166 (2020). RNA can be extracted from a cell of interest using guanidinium thiocyanate lysis followed by CsCl centrifugation, a silica gel-based column (e.g., RNeasy (Qiagen, Valencia, Calif.) or StrataPrep (Stratagene, La Jolla, Calif.)), or using phenol and chloroform, as known in the art. Poly(A)+ RNA can be selected, e.g., by selection with oligo-dT cellulose or, alternatively, by oligo-dT primed reverse transcription of total cellular RNA. RNA can be fragmented by methods known in the art, e.g., by incubation with ZnCl2, to generate fragments of RNA.

In one aspect, total RNA, mRNAs, or nucleic acids derived therefrom (such as cDNA), are isolated from a sample taken from a patient having a predetermined disease. Transcripts that are poorly expressed may be enriched using amplification techniques known in the art.

As described above, the biomarker polynucleotides can be detectably labeled at one or more nucleotides. Any method known in the art may be used to label the target polynucleotides. Preferably, this labeling incorporates the label uniformly along the length of the RNA, and more preferably, the labeling is carried out at a high degree of efficiency. For example, polynucleotides can be labeled by oligo-dT primed reverse transcription. Random primers (e.g., 9-mers) can be used in reverse transcription to uniformly incorporate labeled nucleotides over the full length of the polynucleotides. Alternatively, random primers may be used in conjunction with PCR methods or T7 promoter-based in vitro transcription methods in order to amplify polynucleotides.

The detectable label may be a luminescent label. For example, fluorescent labels, bioluminescent labels, chemiluminescent labels, and colorimetric labels may be used in the practice of the invention. Fluorescent labels that can be used include, but are not limited to, fluorescein, a phosphor, a rhodamine, or a polymethine dye derivative. Additionally, commercially available fluorescent labels including, but not limited to, fluorescent phosphoramidites such as FluorePrime (Amersham Pharmacia, Piscataway, N.J.), Fluoredite (Miilipore, Bedford, Mass.), FAM (ABI, Foster City, Calif.), and Cy3 or Cy5 (Amersham Pharmacia, Piscataway, N.J.) can be used. Alternatively, the detectable label can be a radiolabeled nucleotide.

Nucleic acid hybridization and wash conditions are chosen so that the target polynucleotide molecules specifically bind or specifically hybridize to the complementary polynucleotide sequences of the array, preferably to a specific array site, wherein its complementary DNA is located. Arrays containing double-stranded probe DNA situated thereon are preferably subjected to denaturing conditions to render the DNA single-stranded prior to contacting with the target polynucleotide molecules. Arrays containing single-stranded probe DNA (e.g., synthetic oligodeoxyribonucleic acids) may need to be denatured prior to contacting with the target polynucleotide molecules, e.g., to remove hairpins or dimers which form due to self-complementary sequences.

Optimal hybridization conditions will depend on the length (e.g., oligomer versus polynucleotide greater than 200 bases) and type (e.g., RNA, or DNA) of probe and target nucleic acids. One of skill in the art will appreciate that as the oligonucleotides become shorter, it may become necessary to adjust their length to achieve a relatively uniform melting temperature for satisfactory hybridization results. General parameters for specific (i.e., stringent) hybridization conditions for nucleic acids are described in Sambrook, et al., Molecular Cloning: A Laboratory Manual (3rd Edition, 2001) . Typical hybridization conditions for the cDNA microarrays of Schena et al. are hybridization in 5×SSC plus 0.2% SDS at 65° C. for four hours, followed by washes at 25° C. in low stringency wash buffer (1×SSC plus 0.2% SDS), followed by 10 minutes at 25° C. in higher stringency wash buffer (0.1×SSC plus 0.2% SDS). Particularly preferred hybridization conditions include hybridization at a temperature at or near the mean melting temperature of the probes (e.g., within 51° C., more preferably within 21° C.) in 1 MNaCl, 50 mM MES buffer (pH 6.5), 0.5% sodium sarcosine and 30% formamide.

As discussed above, many RNA sequencing-based methods are available. Non-limiting examples comprise, e.g. whole transcriptome RNA seq, targeted RNA seq, single cell RNA seq, total RNA sequencing, mRNA sequencing, whole transcript RNA sequencing, 3' RNA sequencing, long RNA sequencing, direct RNA sequencing. Each of these sequencing technologies have their own way of preparing samples prior to the actual sequencing step. Depending on the sequencing technology used, amplification steps may be omitted.

As disclosed herein, the differential transcription and/or expression and/or activity level of the gene panel corresponds to a differential expression of, e.g. the transcripts of the whole or part of the transcriptome. In a preferred aspect, the differential transcription and/or expression and/or activity level of the gene panel corresponds to a differential expression of at least one gene of Table 1, more preferably at least 5, at least 7, at least 10 genes selected from Table 1.

In one aspect, the transcription and/or expression and/or activity level of the gene panel corresponds to a downregulated expression of at least one gene of **Table 1,** more preferably at least 5, at least 7, at least 10 genes selected from **Table 1.**

Preferably, the downregulated transcription and/or expression and/or activity of said gene panel corresponds to a decrease equal or superior to about 5 %, preferably equal or superior to about 20 %, more preferably equal or superior to about 40 %, most preferably equal or superior to about 60 %, more preferably equal or superior to about 500%, even more preferably equal or superior to about 1000 %, in particular equal or superior to about 5000 % when compared to the level of corresponding transcription and/or expression and/or activity level of the gene panel determined previously.

In one aspect, the transcription and/or expression and/or activity level of the gene panel corresponds to an upregulated expression of at least one gene of **Table 1,** more preferably at least 5, at least 7, at least 10 genes selected from **Table 1.**

Preferably, the upregulated transcription and/or expression and/or activity of said gene panel corresponds to an increase equal or superior to about 5 %, preferably equal or superior to about 20 %, more preferably equal or superior to about 40 %, most preferably equal or superior to about 60 %, more preferably equal or superior to about 500%, even more preferably equal or superior to about 1000 %, in particular equal or superior to about 5000 % when compared to the level of corresponding transcription and/or expression and/or activity level of the gene panel determined previously.

In one aspect, the transcription and/or expression and/or activity level of the gene panel corresponds to an upregulated expression of at least one gene of **Table 1,** more preferably at least 5, at least 7, at least 10 genes selected from **Table 1** and a downregulated expression of of at least one gene of Table 1, more preferably at least 5, at least 7, at least 10 genes selected from **Table 1.**

As defined herein, patients were classified as responder (CB) or non-responder (N-CB). Patients were considered to have clinical benefit (responder, CB) if they had a clinical progression-free survival (PFS) exhibiting both complete and/or partial response for a predefined period of time from treatment initiation, for example of at least 6 months. Conversely, patients with PFS lower than six months showing stable disease or progression in disease were classified as having no clinical benefit (non-responder, N-CB).

In some cases the differential transcription and/or expression and/or activity level of the gene panel is expressed as a score

The present disclosure recognizes that there are multiple ways to generate a score. In some aspect, this score is preferably a probability score based on trained algorithm.

Usually, such algorithms are trained using expression metric from patients having the same disease as the patient and treated with ICBT and for which response status (CB or N-CB) is known.

In one aspect, a probability score above a determined cutoff value indicates that the patient is determined as responsive, or will respond, to a treatment based on immune checkpoint blockade therapy or treatment (ICBT), or

In one aspect, a probability score below a determined cutoff value indicates that the patient is determined as non responsive, or will not respond, to a treatment based on immune checkpoint blockade therapy or treatment (ICBT).

For example, the algorithm outputs a probability score from 0-1 used to classify the patient(s) as CB or N-CB. By way of example, if the probability score is greater or equal to a determined cutoff value, the patient is classified as CB. In contrast, if the probability score is less than the determined cutoff value, the patient is classified as non-CB.

The person skilled in the art recognizes that there are multiple ways to output the algorithm's score and to determine a cutoff value. Other examples include but are not limited to threshold scores derived from non-probabilistic metrics and empirical measures based on information extracted from training data, dynamically adjusted thresholds responsive to data characteristics or operational context, and multi-dimensional criteria combining multiple metrics for decision-making.

For example, if the probability score is greater or equal to a determined cutoff value 0.5 ±0.2, the patient is classified as CB. In contrast, if the probaility score is less than the determined cutoff value 0.5 ±0.2, the patient is classified as non-CB. Alternatively, for example, the score can be scaled to a percentage values from 0 to 100 or to a different numerical scale, and the cutoff will be redefined to ensure classification with acceptable accuracy.

In order to possibly improve the identification of CB and N-CB patients, in particular when the probability score is considered low confidence, the method of the invention further comprises considering the level and/or amount of circulating tumor DNA (ctDNA) determined at one (t1) and/or more time points (t2, t3, ...) in one or more biological samples obtained from said patient having a cancer.

Circulating tumor DNA (ctDNA) refers to DNA that is released into the circulatory system from tumor cell, for example after apoptosis of the cell, and thus harbours the genetic signatures of the tumor cell, as such, can be qualified, quantitated and traced. CtDNA can be distinguished from non-tumor cell-free DNA (cfDNA) via the detection of mutations that are not found in normal cells, and accounting for clonal hematopoiesis of unknown potential (CHiP). Typically, ctDNA comprises strands of < 145 bp in length and may be highly fragmented. CtDNA measurement has emerged as a method to monitor treatment response. The ctDNA level correlates with tumor burden and can, therefore, be used as a non-invasive tool to monitor treatment response.

Usually, the method comprises a step of extracting circulating free DNA (cfDNA) and determining the level or fraction of ctDNA in one or more biological sample(s).

In one aspect, the biological sample(s) is the same as the biological sample used to determine the level of transcription and/or expression and/or activity of at least one gene of the invention.

In one aspect, the biological sample(s) can be the same but analytes (DNA, RNA) are extracted from different fractions. For example for a blood biological sample, DNA is extracted from plasma and RNA from selected blood cells.

In one aspect, the biological sample(s) used to determine the level and/or amount of ctDNAs is not the same as the biological sample used to determine the level of transcription and/or expression and/or activity of at least one gene of the invention.

In one aspect, the determination of ctDNA dynamic changes comprises determining the level and/or amount of ctDNAs at one (t1) and/or more timepoints (t2, t3, ...). Typically, the level and/or amount of ctDNAs can be expressed at different time points, e.g., as ctDNA copies/mL of whole blood or a fractional component thereof such as, e.g., plasma or serum.

In one aspect, the determination of ctDNA dynamic changes also comprises determining the presence and/or level of genetic signatures of tumor cells such as, e.g., Single nucleotide polymorphisms (SNPs), insertion/deletion polymorphisms (in/dels), and chromosomal rearrangements. These tumor genetic signatures are not only indicative of whether the DNA is derived from tumor cells (tumor DNA) but their presence can also serve as an indicator of ctDNA dynamic changes.

In some aspects, a biological sample is obtained from a patient and then ctDNA is isolated at t1 before a line of treatment is administered (e.g. ICBT).

In some aspects, the level of ctDNA is measured and establishes the baseline ctDNA level at t1 before a line of treatment is administered, e.g. ICBT. In some aspects, the baseline ctDNA level has been determined previously from other patients and the level(s) of ctDNA measured after is/are compared to said baseline.

In some aspects, one or more biological samples are obtained from a patient following one or more doses of the ICBT (at t2, t3, etc...) and the overall level of ctDNA in each such sample is determined. The differential level and/or amount of ctDNAs between the baseline ctDNA level and/or amount (t1) and the subsequent post-ICBT ctDNA level(s) and/or amount(s) (t2, t3, etc...) constitute the ctDNA metric. The ctDNA metric may be calculated as the mean, or the median, or the ratio or the sum, or the weighted mean, median or the sum, the ratio of the level and/or amount of ctDNAs in control samples (e.g. reference samples, baseline, ...) and disease samples.

In one aspect, these times points (t1, t2, t3, *etc*...) correspond to the same time points as those used for determining the level of transcription and/or expression and/or activity of the genes , preferably those selected from **Table 1.**

In another aspect, these times points (t1, t2, t3, *etc*...) do not correspond to the same time points as those used for determining the level of transcription and/or expression and/or activity of the genes, preferably thoseselected from **Table 1.**

Typically, the level and/or amount of ctDNAs is determined at t2, t3, ... in the biological sample between about 1 to about 16 weeks, about 2 to about 14 weeks, about 2 to about 12 weeks, about 2 to about 10 weeks, after the treatment based on ICBT has started.

In one aspect, the level and/or amount of ctDNAs are determined in the same biological samples used to determine the level of transcription and/or activity of the at least gene or gene panel from Table 1 (e.g. whole blood/whole blood, plasma/plasma).

In one aspect, the level and/or amount of ctDNAs are determined in a different biological samples used to determine the level of transcription and/or activity of the at least gene or gene panel from Table 1, e.g. gene panel (e.g. while blood/tumor tissue).

The level and/or amount of ctDNAs of the invention can be detected and, alternatively, quantitated by a variety of sequencing methods including, but not limited to, microarray analysis, polymerase chain reaction (PCR), digital PCR, serial analysis of gene expression (SAGE), immunoassay, and mass spectrometry.

In an aspect of the invention, the amount and/or level of ctDNA to be used for subsequent sequencing detection is not sufficient and needs to be amplified using conventional methods or techniques described, e.g. in US 20170067117 (HAPLOX BIOTECHNOLOGY).

In one aspect, the amount and/or level of ctDNA were determined at one timepoint and classified into low or high ctDNA content.

In one aspect, on-treatment changes in the amount and/or level of ctDNA were dichotomized into increase versus no increase, based on changes in, e.g. ctDNA copies/mL of blood, e.g. whole blood or plasma. Patients with an increase were predicted not to respond (N-CB), whereas patients without an increase during treatment were predicted to respond (CB). Patients with undetectable ctDNA in both the baseline and on-treatment sample were categorized as no increase/predicted-CB since low baseline ctDNA levels are considered a prognostically favorable sign.

Where the probability score is high confidence, the patient is determined as responsive (responder, CB) or not responsive (non-responder, N-CB) to said treatment based on this sole gene panel's probability score. In one aspect, the term "high confidence" means that the probability score lies outside an interval defined as the cutoff value ± uncertainty value, for example if cutoff is 0.5 and uncertainty value is ±0.1, a score < 0.4 or > 0.6 is high confidence.

Where the probability score is low confidence, then the differential level and/or amount of ctDNAs , and in particular the determined ctDNA metric is also considered to determine the patient's response to treatment.In one aspect, the term "low confidence" means that the probability score lies inside an interval defined as the cutoff value ± uncertainty value, for example if cutoff is 0.5 and uncertainty value is ±0.1, a score >= 0.4 or <= 0.6 is low confidence.

Where the probability score and the ctDNA metric are low confidence, then the probability score or the differential level and/or amount of ctDNAs is considered to determine whether the patient is responsive (responder, having clinical benefit (CB)) or not responsive (non-responder, patient not having clinical benefit (N-CB)) to said treatment.

The ctDNA metric is considered to predict the patient's response to treatment if the ctDNA metric (e.g. ctDNA ratio) is of high confidence.

For example, if the probability score is < 0.4±0.1 or > 0.6±0.1, the probability score is considered high confidence and used to classify the patient:
a. If probability score >= 0.5±0.2 classify as CB
b. If probability score< 0.5 ±0.2 classify as non-CB

For example, if the probability score is >= 0.4±0.1 and <= 0.6±0.1 and the ctDNA metric is either <= 0.5±0.4 or >= 1.5±0.4, the ctDNA metric is used to classify the patient:
a. ctDNA metric <= 1±0.9 the patient is classified as CB
b. ctDNA metric >= 1±0.9 the patient is classified as non-CB

For example, if the probability score is >= 0.4±0.1 and <= 0.6±0.1 and the ctDNA metric is > 0.5±0.4 and < 1.5±0.4, the probability score is used to classify the patient:
a. If probability score >= 0.5 ±0.2 the patient is classified as CB
b. If probability score < 0.5 ±0.2 the patient is classified as non-CB

According to an aspect of the method described herein, if the patient having a cancer is determined as responsive (classified as responder (CB)), or predicted to respond to a treatment based on immune checkpoint blockade therapy or treatment (ICBT), the treatment is continued.

According to an aspect of the method described herein, if the patient having a cancer is determined as responsive (classified as responder (CB)) to a treatment based on immune checkpoint blockade therapy or treatment (ICBT) and the treatment has not yet started, the treatment is started.

According to an aspect of the method described herein, if the patient having a cancer is determined as not responsive (classified as non-responder (N-CB)) to a treatment based on immune checkpoint blockade therapy or treatment (ICBT), the method further comprises a step of adapting the treatment.

Adapting the treatment comprises not administering the envisioned treatment or inhibitor and/or further administering a combination or additional therapy, and/or adapting the dose, amount and/or regimen, e.g. the treatment, such as e.g. the treatment based on immune checkpoint blockade therapy or treatment (ICBT) described herein.

Usually, the combination or additional therapy is selected from the group comprising chemotherapy, immunotherapy, hormonotherapy, targeted therapy (such as e.g. Tyrosine Kinase Inhibitor), gene or cell therapy, or radiotherapy or a combination of one or more thereof.

The invention also provides a method for predicting and/or determining if a patient having a cancer will respond, or is responsive, to a treatment based on immune checkpoint blockade therapy or treatment (ICBT), said method comprising determining, in one or more biological samples obtained from said patient having a cancer,
i) the level of transcription and/or expression and/or activity of a gene panel known to predict the likelihood to respond to a treatment based on immune checkpoint blockade therapy or treatment (ICBT), and
ii) expressing said differential transcription and/or expression and/or activity level as a gene expression metric,
iii) classifying the samples according to the gene expression metric using an algorithm trained on expression data obtained from samples labelled as responder(s) (CB) and non-responder(s) (N-CB) and, optionally, selecting a gene panel comprising at least one gene responding to treatment based on immune checkpoint blockade therapy or treatment (ICBT),
iv) determining at one (t1) or more time points (t2, t3, ...) in one or more biological samples obtained from said patient having a cancer, the level and/or amount of circulating tumor DNA (ctDNA),
v) determining a differential level and/or amount of ctDNAs by comparing the level and/or amount at one (11) or more time points (t2, t3, ...) versus the level and/or amount determined previously, and

wherein differential transcription and/or expression and/or activity level of the genes, or the gene panel, relative to the level of corresponding transcription and/or expression and/or activity level of the genes or gene panel determined previously, and
wherein differential level and/or amount of ctDNAs, in the biological sample, determined at one or more time points (t2, t3, ...) after starting a treatment based on ICBT relative to corresponding level and/or amount of ctDNAs determined previously,
are predictive of the patient's response to said treatment.

The invention also provides a method for predicting and/or determining if a patient having a cancer will respond, or is responsive, to a treatment based on immune checkpoint blockade therapy or treatment (ICBT), said method comprising,
i) determining at one (t1) or more time points (t2, t3, ...) the level of transcription and/or expression and/or activity of at least one gene in one or more biological samples obtained from said patient having a cancer,
ii) determining a differential transcription and/or expression and/or activity level of the at least one gene by comparing the transcription and/or expression and/or activity level at one (11) or more time points (t2, t3, ...) versus the transcription and/or expression and/or activity level determined previously,
iii) expressing said differential transcription and/or expression and/or activity level as a gene expression metric,
iv) classifying the samples according to the gene expression metric using an algorithm trained on expression data obtained from samples labelled as responder(s) (CB) and non-responder(s) (N-CB) and, optionally, selecting a gene panel comprising at least one gene responding to treatment based on immune checkpoint blockade therapy or treatment (ICBT),
v) determining at one (11) or more time points (t2, t3, ...) in one or more biological samples obtained from said patient having a cancer, the level and/or amount of circulating tumor DNA (ctDNA),
vi) determining a differential level and/or amount of ctDNAs by comparing the level and/or amount at one (11) or more time points (t2, t3, ...) versus the level and/or amount determined previously, and

wherein differential transcription and/or expression and/or activity level of the genes, or the gene panel, relative to the level of corresponding transcription and/or expression and/or activity level of the genes or gene panel determined previously, and
wherein differential level and/or amount of ctDNAs, in the biological sample, determined at one or more time points (t2, t3, ...) after starting a treatment based on ICBT relative to corresponding level and/or amount of ctDNAs determined previously,
are predictive of the patient's response to said treatment.

The invention also provides a computer-implemented method for implementing a method for predicting or determining if a patient having a cancer will respond, or is responsive, to a treatment based on immune checkpoint blockade therapy or treatment (ICBT), said computer-implemented method comprising:
i) performing RNA sequencing on at least one biological sample obtained from said patient having a cancer, to determine at one or more time points in at least one or more biological samples obtained from said patient having a cancer, the level of transcription and/or expression and/or activity of a gene panel of at least 5 genes, at least 7 genes, at least 10 genes selected from **Table 1,**
ii) expressing the differential transcription and/or expression and/or activity level of the gene panel as a gene expression metric,
iii) inputting the gene expression metric into an algorithm that was trained using the same gene expression metric obtained from patients having cancer, preferably the same cancer, and treated with a treatment based on ICBT and for which response status (responder (CB) or non-responder (N-CB)) is known,
iv) outputting a score, preferably a probability score.
v) analysing DNA sequencing from circulating free DNA (cfDNA) obtained from at least one biological sample of the patient,
vi) determining the ctDNA level and/or amount and/or metric in the at least one biological sample of the patient,
vii) If the score of iv) is high confidence, the patient is determined as responsive or non-responsive to said treatment based on the score determined at step iv).

If the score of iv) is low confidence and the ctDNA metric of vi) is high confidence, the patient is determined as responsive or non-responsive to said treatment based on the ctDNA metric determined at step vi).

If both the score determined at step iv) and the ctDNA metric determined in step vi) are considered low confidence, the patient is determined as responsive or non-responsive to said treatment based on the score determined at step iv). Alternatively, if both the score determined at step iv) and the ctDNA metric determined in step vi) are considered low confidence, the patient is determined as responsive or non-responsive to said treatment based on the ctDNA metric determined at step vi).

Alternatively, steps v) to vi), or v) to vii) are performed directly after step iv) or in parallel to steps i) to iii) or i) to iv).

Also encompassed in the present invention is the use of a method of the invention.

Also encompassed in the present invention is the use of at least one gene, at least 5 genes, at least 7 genes, at least 10 genes selected from Table 1 in a method for predicting or determining if a patient having a cancer will respond, or is responsive, to a treatment based on ICBT.

Also encompassed in the present invention is a gene panel, comprising, or consisting of, RAD51, SCARF1, SMPD3, CLC, CD86, PDXK, PTTG1, TOX, CEBPA and ENSG00000279447.

Also encompassed in the present invention are methods of treatment and/or prevention of cancer. In one aspect, the method of treatment and/or prevention comprises:
performing the method for predicting or determining if a patient having a cancer will respond, or is responsive, to a treatment based on ICBT as disclosed herein; and
i) if the patient having a cancer is determined as responsive (responder, CB) to said treatment based on ICBT, the treatment is continued, or
ii) if the patient having a cancer is determined as not responsive (non-responder, N-CB) to said treatment based on ICBT, the method further comprises a step of adapting the treatment.

Adapting the treatment comprises not administering the envisioned treatment or inhibitor and/or further administering a combination or additional therapy, and/or adapting the dose, amount and/or regimen, e.g. the treatment, such as e.g. the treatment based on immune checkpoint blockade therapy or treatment (ICBT) described herein.

Further encompassed in the present invention is a kit for performing a method of the invention, said kit comprising a) means and/or reagents for determining the level of transcription and/or expression and/or activity of said gene panel in a biological sample from said patient, and b) instructions for use.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein. Various references are cited throughout this Specification, each of which is incorporated herein by reference in its entirety. The foregoing description will be more fully understood with reference to the following Examples.

### EXAMPLES

### Example 1

### Materials and Methods

### Sex as a biological variable

Both female and male patients were included. In this study, sex was not considered as a biological variable.

### Patients

This Dutch, multicenter study included 93 patients with mUC who were treated with anti-PD-(L)1 between 2017 and 2023. Patients were treated with nivolumab or pembrolizumab in the first- or second-line mUC setting or with maintenance avelumab following response or stable disease to platinum-based chemotherapy. Patients with measurable disease were evaluated according to RECIST1.1 (29). Clinical endpoint was clinical benefit (CB) at 6 months, defined as radiological and clinical progression-free survival (PFS) at 6 months.

### Blood Collection and Processing

Blood was drawn prior to the first three cycles of anti-PD-(L)1 therapy (i.e., at 0, 2 and 4 weeks for nivolumab and avelumab and at 0, 3 and 6 weeks for pembrolizumab). At these timepoints, a complete blood cell count was performed as part of routine clinical care. In addition, blood was collected in a PAXgene Blood RNA tube for whole blood RNA analyses (BD Biosciences, San Jose, CA, USA) and in three 10 mL EDTA or cell-free DNA (cfDNA) collection tubes (Roche) for ctDNA analyses. PAXgene tubes were stored at -80°C until RNA purification. EDTA and cfDNA tubes were processed as previously described (25). The baseline sample and the earliest on-treatment sample available were used for analyses.

### TMB and PD-L1

Tumor tissue for molecular analysis and PD-L1 staining was obtained from diagnostic biopsies obtained in routine clinical practice. The PD-L1 staining was performed on formalin-fixed paraffin-embedded (FFPE) tissue sections using antibodies against 22C3 or E1L3N (Cell Signalling). A combined positivity score (CPS) was calculated by dividing the number of stained cells expressing PD-L1 (tumor cells, tumor-associated lymphocytes and macrophages) by the total number of viable tumor cells, multiplied by 100, taking into account at least 200 viable tumor cells, not adjacent to necrotic areas. A CPS≥10 was considered positive. Tumor sequencing data were generated utilizing different sequencing platforms: whole genome sequencing (WGS), whole exome sequencing (WES), TruSight Oncology 500 (TSO500), Foundation Medicine T7 assay (CLIA: 22D2027531), single molecule Molecular Inversion Probe panel (PATHv3D) and/or the ctDNA_NGSv1 targeted sequencing panel (25). WGS, WES and TSO500 data were used to determine non-synonymous tumor mutational burden (nsTMB). A nsTMB>10 mutations per megabase was considered high.

### ctDNA

ctDNA analyses were performed in 88 patients, of which 53 patients were included in a prior publication (25). Only patients with paired baseline and on-treatment samples who were evaluable for the clinical endpoint were included in the current analyses.

ctDNA workup and downstream analysis were performed as previously described (25). In short, cell-free DNA (cfDNA) was isolated from blood plasma (median 5.6 mL, IQR 5 mL - 8 mL) using the QIAamp Circulating Nucleic Acid kit (Qiagen). White blood cell (WBC) DNA was isolated using a QIAamp DNA Mini Kit (Qiagen). A maximum of 50 ng cfDNA ng (median 50 ng, IQR 37 ng - 50 ng) and 50-80 ng of mechanically sheared WBC DNA were used for targeted sequencing using an in-house developed and validated 117 kb targeted sequencing panel (NEN-EN-ISO 15189+C11:2015) (25). Libraries were generated using the TWIST Library Preparation Kit (TWIST Biosciences) in combination with xGen dual index unique molecular identifiers (UMI) adapters (Integrated DNA Technologies) or TWIST UMI adapters (TWIST Biosciences). Libraries were paired-end sequenced on a NovaSeq6000 platform (Illumina). Reads were mapped to hg19 and deduplicated using the read-specific UMI information (Fgbio). Unique reads that not met fgbio quality parameters and/or based on <2 UMI reads (singletons) were only kept for variant detection in *TERT* protomer region and copy number variant (CNV) detection.

Somatic variants were called using Genomic Analysis Toolkit (GATK) Mutect2 (version 4.1.5.0) based on previously described filter criteria (25). Variants with at least 5 supporting variant reads and >0.1% variant allele fraction (VAF) were selected for downstream analysis. Additionally, patient-specific cfDNA variants and, if available, tumor variants (evaluation of nonsynonymous tumor variants with a minimal read depth of 10, N=59 pts), were evaluated in the patient-matched baseline (BL) and on-treatment (OT) cfDNA sequencing data. For this dependent calling, the variant in the matched BL or OT sample had to be supported by at least 3 variant reads, the VAF signal had to be at least 20x higher than the average VAF of 22 control cfDNA samples and at least three times higher than the patient-matched WBC sample for that specific nucleotide change (if available).

CNV detection was performed as previously described using both the relative coverage and the median allele fraction (MAF) divergence from heterozygosity. Copy number loss was defined as relative coverage ≤ -0.3 or relative coverage ≤ -0.1 and MAF ≥ 0.6. Copy number gain was defined as a relative coverage ≥ 0.3 or ≥ 0.1 and MAF ≥ 0.6.

CtDNA fraction was determined by using the somatic mutation with highest VAF in a non-amplified region corrected for loss of heterozygosity (LOH) or using the MAF deviation from heterozygosity of germline single nucleotide polymorphisms (SNPs) in genes with a single-copy loss (25,30). CtDNA fractions were converted to ctDNA copies per mL plasma (total cfDNA concentration multiplied by 303). To incorporate technical uncertainty and biological variability of ctDNA levels, lower and higher limits were estimated as previously described (25).

On-treatment changes were dichotomized into increase versus no increase, based on changes in ctDNA copies/mL. Patients with an increase were predicted to not have CB (N-CB), whereas patients without an increase during treatment were predicted to have CB. Patients with undetectable ctDNA in both the baseline and on-treatment sample were categorized as no increase/predicted-CB since low baseline ctDNA levels are considered a prognostically favorable sign (31). CtDNA-based specificity and sensitivity was calculated for the full ctDNA cohort.

### Whole Blood RNA Sequencing

Whole blood RNA sequencing was performed in 79 patients with paired samples. Additionally, 2 patients with on-treatment samples only were used for differential gene expression analysis (DEA). Total RNA was extracted from whole blood using the PAXgene blood miRNA kit (Qiagen, Venlo, Netherlands). RNA quantity was determined using Qubit (Thermo Fisher Scientific, Waltham, MA, USA). RNA quality was assessed on a Tapestation 4,200 (Agilent Technologies, Santa Clara, CA, USA). Per sample, at least 200 ng of total RNA was used for library preparation. RNA samples were treated for globin RNA depletion with the QIAseq FastSelect RNA Removal kit (Qiagen, Venlo, Netherlands). Library preparation was performed after isolation of poly-A RNA by means of NEBNext poly(A) mRNA magnetic isolation module and then, setup of directional RNA libraries by means of NEBNext Ultra II directional RNA library prep kit in combination with NEBNext multiplex oligos for Illumina Set 1, Set 2 and Set 3 was performed (NEB, Ipswich, MA, USA). Library quality control was done by using Dual AmpureXP cleanup for complete adapter dimer removal, and a verification of adapter dimer removal with TapeStation 4,200 (Agilent Technologies, Santa Clara, CA, USA).

All Libraries were pooled by equal volume and a test sequencing run was done on iSeq100 (Illumina, San Diego, CA, USA) to determine content of each library and adjust the final pool. Sequencing was performed on Illumina NovaSeq6000, 3 lanes of S4 flow cell, Paired-End 150 configuration with an expected output of 800Gb per lane or ca. 2,600M PE reads per lane (Illumina, Sain Diego, CA, USA). A minimum of 30M PE150 reads were required per sample. The FastQ files with paired-end reads were used as input for gene expression analysis on the LITOSeek^{®} platform (Novigenix SA, Epalinges, Switzerland). Of note, whole blood RNA sequencing data of patients in the discovery cohort has been previously published (32). Samples have been re-sequenced and re-analyzed for this paper after optimization of the analyses pipeline.

### Data processing and Quality Check

Sequence data quality was evaluated using FastQC (version 0.11.9) combined with MultiQC (version 1.11). Cutadapt (3.4) was used to find and remove adapter sequences, primers, poly-A tails and other types of unwanted sequence from high-throughput sequencing. Reads were aligned to the Human genome assembly (GRCh38) along with its corresponding annotation from Ensembl database using the release 107. The pseudo-alignment and quantification of transcript abundance of the RNA-Seq reads was done using Salmon (version 1.5.2) with default parameters. All samples were used for downstream analysis.

### Data transformation and exploratory analysis

Normalization for gene length, Transcripts Per Million (TPM) values, was conducted as a step downstream in our analysis. Gene pseudo-counts from Salmon were imported into the R statistical computing environment (version 4.2.1) and subsequently filtered by excluding genes with less than 1 count per million (CPM) across all samples and with a coefficient of variance (cv) of 100, using the filtered.data function within the NOISeq R package (version 2.40.0). Following the initial gene data treatment, forward normalization was performed employing the variance-stabilizing transformation using the vst function, which is a feature of the DeSeq2 R package (version 1.36.0). Primary focus for exploratory data analysis centered on the vst-transformed values and the selected subset of genes from NOISeq. Principal Component Analysis (PCA) and scatter plots were applied to visualize the similarities and differences among samples.

### Differential gene expression and multivariate analysis

Comprehensive analysis of differential gene expression was performed using proprietary algorithms and curation of the differentially expressed genes (DEGs). Three DEAs were performed: the first compared the on-treatment (OT) samples of patients with CB with their paired baseline (BL) samples, the second compared the OT and BL samples of patients with N-CB and the third compared the OT samples of patients with CB versus patients with N-CB. Functional and network analyses of the DEGs were realized with STRING (version 12.0) and Cluster-Profiler (version 4.6.2) to perform over-representation analysis (ORA), which allowed to identify central biological pathways and biomarkers of response. STRING clusters were defined by MCL clustering (inflation parameter=3) on the STRING online platform. Significantly enriched ORA pathways were defined by an adjusted p-value≤0.05 (Benjamini-Hochberg method). Additionally, the DEGs attributed to any enriched terms from the ORA results output were extrapolated, enabling identification of the functionally relevant genes among all DEGs. Basic plots were performed with RStudio (version 4.2.1) and the correspondent R packages ggplot2 (version 3.5.0), UpSetR (version1.4.0). Heatmaps were generated with ComplexHeatmap (version 2.14.0), ROCR (version 1.0-11) was used to plot ROC curves, survminer (version 0.4.9) and survival (version 3.5-8) were used to generate Kaplan Meier curves for PFS.

### Modeling

For response prediction modeling, patients were distributed in discovery, test and validation cohorts, based on the timing of enrollment and sample collection. The discovery cohort was used for biomarker discovery and model training, the test cohort for independent model testing and selection and the validation cohort for final blinded validation of the model. We employed the SPLS (Sparse Partial Least Squares) method, which is particularly effective for small sample sizes and enhances model interpretability. The modeling process incorporated a resampling method, repeated cross-validation with 10 iterations and a repeated k-fold cross-validation of 3 for the discovery dataset.

Model's performance was assessed by plotting the True Positive Rate (TPR) against the False Positive Rate (FPR) at sensitivity and specificity thresholds of 90%. To classify samples as CB or N-CB, a 55% probability cut-off was used. The efficiency of the model was further verified by plotting Kaplan-Meier survival curves based on the model's predictions, along with the corresponding hazard ratios and the distance between predictive curves of responders and non-responders at 50% of the PFS.

Feature reduction was performed during the modeling process based on the initial feature lists to identify the optimal model based on AUC performance. This reduction was systematically applied by specifying feature selection within the ranges of 10, 15, and 20 features.

### Multimodal modeling

The multimodal model was optimized on the test cohort and blindly validated on the validation cohort. We did not use the discovery cohort (where the RNA model was trained) to avoid multimodal model overfitting. Specifically, for the development of a multimodal model based on RNA and ctDNA, RNA model prediction probabilities and ctDNA ratio values (ctDNA copies/mL at OT / ctDNA copies/mL at BL) were incorporated in the test cohort for thresholds optimization. Specifically, ctDNA-based predictions were adjusted using the RNA model prediction if a patient's ctDNA ratio value fell within an uncertainty range around the ctDNA ratio cutoff= 1. Multiple multimodal models were created by enlarging this uncertainty range, and by applying different RNA model prediction probabilities cutoffs. Each multimodal model performance was then assessed by calculating specificity and sensitivity, which was then compared to the standalone RNA model performance. The best combination of ctDNA ratio and predictive probability cutoffs was selected in the test cohort, allowing for the highest increase in sensitivity and specificity compared to the standalone RNA model performance. Cutoffs were then applied to the validation cohort.

### Statistics

Nonparametric data were analyzed by a Wilcoxon test (paired or unpaired depending on the experimental setup). P < 0.05 was considered as statistically significant. Differences in PFS in Kaplan-Meier curves were assessed by Mantel-Haenszel test. Each specific statistical test is reported for each experiment in the figure legends. Boxplots are used to present the data, showing median and the 25th to 75th percentiles.

### Study approval

The study was conducted in accordance with relevant guidelines and regulations, and approved by the CMO Radboudumc local medical ethics committee (local registration numbers 2016-3060 and 2020-6778). Written consent was obtained from all patients for the use of biomaterials.

### Results

### Clinical characteristics of the ICI-treated mUC patient cohort

To longitudinally and non-invasively monitor response to ICI and to discover biomarkers predictive of CB, we collected blood liquid biopsies (LBx) from a total of 93 mUC patients treated with either pembrolizumab (N=72), nivolumab (N=7) or avelumab (N=14) (Figure 1A). Specifically, baseline (BL) blood LBx were collected before ICI therapy initiation, while on-treatment (OT) LBx were collected after cycle 1 or 2 (2-6 weeks). LBx samples were used for ctDNA and bulk whole blood RNA sequencing (RNA-seq) analysis (see material and methods section for details). We collected paired BL and OT ctDNA data for 88 patients and RNA-seq data for 79 patients, of whom 74 patients had both ctDNA and RNA data. Moreover, archival tumor tissue (FFPE) was used to determine PD-L1 CPS (N=62) and tumor mutational burden (TMB) (N=78). Of note, for RNA-seq analysis and modeling, patients were distributed in separate discovery, testing and validation cohorts for optimal data analysis. Clinical endpoint was clinical benefit at 6 months, defined as radiological and clinical PFS at or beyond 6 months from treatment initiation. Out of the 93 patients included, 42 patients experienced clinical benefit (CB) and 51 did not (N-CB).

### CtDNA profiling outperforms conventional tumor biomarkers for prediction of N-CB in ICI-treated mUC patients

High TMB and PD-L1 expression in the tumor have previously been associated with CB to ICI (33,34). We, therefore, assessed whether these two tumor biomarkers could help stratify the cohort into patients with or without CB (Figure 1, B and C). High PD-L1 expression was defined as a combined positive score (CPS) ≥ 10, in line with what is used in the clinic to select cisplatin-ineligible mUC patients for first-line ICI. There was a weak trend, but no significant association, between CPS ≥ 10 and longer PFS (Figure 1B). High TMB, defined as a TMB ≥ 10 mutations per megabase, was significantly associated with improved PFS (Figure 1C), but low TMB had only 80% sensitivity and 43% specificity to predict N-CB. These results indicate that conventional tumor biomarkers only have partial ability to predict CB to ICI in mUC and that more accurate biomarkers are needed.

We previously demonstrated that decreases in ctDNA after 3-6 weeks show high specificity and moderate sensitivity for predicting CB to ICIs in a subset of patients of the presented mUC cohort(25). We expanded our previous cohort for the current analyses to a total of 88 patients. Patients were categorized into two groups based on their ctDNA dynamics. Patients with a ctDNA-increase or stable from BL to OT were predicted to have N-CB (N=32), while patients with a ctDNA-decrease from BL to OT (N=45) or undetectable ctDNA at both timepoints (N=11) were predicted to have CB. Patients with predicted CB had significantly longer PFS compared to those with predicted N-CB (Figure 1D). The ctDNA-based model showed 59% sensitivity and 92% specificity to detect N-CB patients (true positive cases). Altogether, these data support the potential of ctDNA to predict N-CB, which outperforms the conventional tumor biomarkers PD-L1 and TMB.

### Immunotranscriptome data and machine-learning (ML) approaches allow to develop robust models for early response prediction to ICI

To develop predictive models of CB to ICI, the biomarkers identified in the discovery cohort were used as input to generate multiple models, using several iterations of biomarker subsets selection (Figure 2A) (see material and methods for details). Subsequently, the best performing immunotranscriptome model (RNA model) was selected based on predictions in the independent test cohort. This final RNA model, comprising 10 genes, was selected based on area under the curve (AUC) ranking of the receiver operating characteristics curve (ROC) for predicting CB to ICI and by ranking the difference in median PFS between the predicted CB and N-CB groups (Figure 2B-C and 3A-B). The RNA model showed high performance in both independent test cohort (73% sensitivity at 79% specificity, AUC=0.84, N=29) and discovery cohort (92% sensitivity at 71% specificity, AUC=0.86, N=29). Interestingly, when using other gene sets derived from Table 1, the performance in the test cohort was inferior to the 10-gene RNA model (Figure 3C). Also, the 10-gene panel did not show any overlap with a selection of literature-based tumor-derived signatures which have been shown to correlate with CB to ICI in other studies (Figure 3D) (26-28), although part of these genes was among the identified biomarkers. Of note, models based on these literature-based signatures showed low performance in our cohorts (Figure 3E). Lastly, we validated the model performance in a small blind validation cohort (N=21). The RNA model achieved an AUC of 0.77 (67% sensitivity at 67% specificity, N=21) (Figure 2D) and there was a non-significant trend towards longer PFS in the predicted CB group (Figure 2E). To summarize, ML-based approaches for biomarker selection and modelling identified the most central RNA biomarkers in whole blood and showed high accuracy in the discovery and testing cohort for predicting CB.

### Multimodal modelling with ctDNA and RNA-based biomarkers boosts model performance and leads to accurate prediction of N-CB in the independent validation cohort

Emerging evidence suggests that combining biomarkers from dissimilar sources can exponentially improve model performance (37,38). We, therefore, hypothesized that we could improve predictions by integrating RNA- and ctDNA-based readouts in one model. When comparing the predictions of the standalone ctDNA- and RNA-based models in the test cohort (Figure 4A), we found that models showed discordant predictions for a significant proportion of patients (12/27). We then reasoned that the fixed cutoffs used in the standalone models might influence wrong readout occurrence. Therefore, we developed a multimodal model in the test cohort where the final prediction was based on a cutoff range of combined ctDNA ratio and RNA-based prediction probability (see material and methods section for details). The multimodal model was then validated in the independent validation cohort. The multimodal model showed superior performance compared to the standalone approaches (Figure 4B), reaching 71% sensitivity at 100% specificity in the test cohort, and 79% sensitivity at 100% specificity in the independent blinded validation cohort. Consequently, the multimodal approach allowed for a superior stratification of the predicted CB and N-CB groups (Figure 4C-E). Thus, this multimodal analysis appears a promising tool for early response prediction to ICI.

### Example 2 - RNA stand-alone prediction

Method to predict ICI response for cancer patient.
1. Collect whole blood samples for a patient at one or more time points;
2. Determine expression level for a panel of genes (preferrably 10) previously determined, based on analysis of expression data from the samples obtained from patients undergoing treatment, to be informative of ICI response;
3. Determine expression metric for each gene in the panel, any metric that reflects a difference (difference or ratio for example) between the expression measured in the two samples;
4. Input gene expression metric for each gene in the panel into a trained algorithm. The algorithm was trained using expression metric from patients having the same disease as the patient and treated with ICI and for which response status (CB or N-CB) is known;
5. Algorithm outputs a probability score (0-1);
6. Classify patient:
   a. If probability score >= 0.5 ±0.2 classify as CB
   b. If probability score < 0.5 ±0.2 classify as non-CB

### Example 3 - Multimodal prediction

Method to predict ICI response for cancer patient.
1. Collect whole blood samples for a patient at one or more time points;
2. Extract RNA and determine expression level for a panel of genes (preferred 10) previously determined, based on analysis of expression data from the samples obtained from patients undergoing treatment, to be informative of ICI response;
3. Determine expression metric for each gene in the panel;
4. Input gene expression metric for gene in the panel into a trained algorithm. The algorithm was trained using expression metric from patients having the same disease as the patient and treated with ICI and for which response status (CB or N-CB) is known;
5. Algorithm outputs a probability score (0-1);
6. Extract circulating free DNA and determine the amount of circulating tumor DNA from whole blood samples collected at different time points from patient;
7. Determine the ration ctDNA between ctDNA amount at t1 and other time point;
8. Input probability score obtained in step 5 and ctDNA metric for patient into classifier.
9. Output classification for patient.

The current implementation of the multimodal classifier is based on a decision tree. The decision tree outputs classification.. If probability score (obtained in step 5) is high confidence, this probability score is used for classification. If probability score is considered low confidence the ctDNA metric( determined in step 7) is used if high confidence to make classification. If both probability score and ctDNA metric are low confidence the classification is done based on probability score (obtained in step 5). The scores used in the implementations are:
For example, if the probability score is < 0.4±0.1 or > 0.6±0.1, the probability score is considered high confidence and used to classify the patient:
   c. If probability score >= 0.5 ±0.2 classify as CB
   d. If probability score< 0.5 ±0.2 classify as non-CB
For example, if the probability score is >= 0.4±0.1 and <= 0.6±0.1 and the ctDNA metric is either <= 0.5±0.4 or >= 1.5±0.4, the ctDNA metric is used to classify the patient:
   c. ctDNA metric <= 1±0.9 the patient is classified as CB
   d. ctDNA metric >= 1±0.9 the patient is classified as non-CB
For example, if the probability score is >= 0.4±0.1 and <= 0.6±0.1 and the ctDNA metric is > 0.5±0.4 and < 1.5±0.4, the probability score is used to classify the patient:
   c. If probability score >= 0.5 ±0.2 the patient is classified as CB
   d. If probability score < 0.5 ±0.2 the patient is classified as non-CB

### Example 4 - Multimodal prediction

Method to predict ICI response for cancer patient.
1. Collect samples for a patient at one time point;
2. Determine the expression metric for a panel of genes (preferrably 10) previously determined, based on analysis of expression data from patients undergoing treatment, to be informative of ICI response;
3. Input gene expression metric for each gene in the panel into a trained algorithm. The algorithm was trained using expression metric from patients having the same disease as the patient and treated with ICI and for which response status (CB or N-CB) is known;
4. Algorithm outputs a probability score (0-1);
5. Determine circulating tumor DNA metric;
6. Input probability score obtained in step 4 and ctDNA metric for patient into classifier;
7. Output classification for patient.

### REFERENCES

1. Hanahan D, Weinberg RA. Hallmarks of cancer: The next generation. Cell [Internet]. 2011 Mar 4 [cited 2024 Dec 16];144(5):646-74. Available from: http://www.cell.com/article/S0092867411001279/fulltext
2. Hanahan D, Weinberg RA. Hallmarks of cancer: The next generation. Cell [Internet]. 2011 Mar 4 [cited 2024 Dec 16];144(5):646-74. Available from: http://www.cell.com/article/50092867411001279/ful Itext
3. Chen DS, Mellman I. Oncology meets immunology: The cancer-immunity cycle. Immunity [Internet]. 2013 Jul 25 [cited 2024 Dec 16];39(1):1-10. Available from: http://www.cell.com/article/S1074761313002963/fulltext
4. FDA Approval Timeline of Active immunotherapies | CRI [Internet]. [cited 2024 Dec 16]. Available from: https://www.cancerresearch.org/regulatory-approval-timeline-of-active-immunotherapies
5. Fountzilas E, Vo HH, Mueller P, Kurzrock R, Tsimberidou AM. Correlation Between Biomarkers and Treatment Outcomes in Diverse Cancers: A Systematic Review and Meta-Analysis of Phase I and II Immunotherapy Clinical Trials. Eur J Cancer [Internet]. 2023 Aug 1 [cited 2024 Dec 16];189:112927. Available from: https://pmc.ncbi.nlm.nih.gov/articles/PMC10528229/
6. Herbst RS, Soria JC, Kowanetz M, Fine GD, Hamid O, Gordon MS, et al. Predictive correlates of response to the anti-PD-L1 antibody MPDL3280A in cancer patients. Nature 2014 515:7528 [Internet]. 2014 Nov 26 [cited 2024 Dec 16];515(7528):563-7. Available from: https://www.nature.com/articles/nature14011
7. Wang MM, Coupland SE, Aittokallio T, Figueiredo CR. Resistance to immune checkpoint therapies by tumour-induced T-cell desertification and exclusion: key mechanisms, prognostication and new therapeutic opportunities. British Journal of Cancer 2023 129:8 [Internet]. 2023 Jul 15 [cited 2024 Dec 16];129(8):1212-24. Available from: https://www.nature.com/articles/s41416-023-02361-4
8. Balar A V., Galsky MD, Rosenberg JE, Powles T, Petrylak DP, Bellmunt J, et al. Atezolizumab as first-line treatment in cisplatin-ineligible patients with locally advanced and metastatic urothelial carcinoma: a single-arm, multicentre, phase 2 trial. Lancet [Internet]. 2017 Jan 7 [cited 2024 Dec 16];389(10064):67-76. Available from: https://pubmed.ncbi.nlm.nih.gov/27939400/
9. Strickler JH, Hanks BA, Khasraw M. Tumor Mutational Burden as a Predictor of Immunotherapy Response: ls More Always Better? Clin Cancer Res [Internet]. 2021 Mar 1 [cited 2024 Dec 16];27(5):1236-41. Available from: https://pubmed.ncbi.nlm.nih.gov/33199494/
10. Zhang W, Kong Y, Li Y, Shi F, Lyu J, Sheng C, et al. Novel Molecular Determinants of Response or Resistance to immune Checkpoint inhibitor Therapies in Melanoma. Front Immunol. 2022 Jan 11;12:798474.
11. Bellmunt J, de Wit R, Vaughn DJ, Fradet Y, Lee JL, Fong L, et al. Pembrolizumab as Second-Line Therapy for Advanced Urothelial Carcinoma. New England Journal of Medicine. 2017 Mar;376(11):1015-26.
12. Powles T, Park SH, Voog E, Caserta C, Valderrama BP, Gurney H, et al. Avelumab Maintenance Therapy for Advanced or Metastatic Urothelial Carcinoma. https://doi.org/101056/NEJMoa2002788 [Internet]. 2020 Sep 18 [cited 2021 Aug 11];383(13):1218-30. Available from: https://www.nejm.org/doi/full/10.1056/NEJMoa2002788
13. Powles T, Bellmunt J, Comperat E, De Santis M, Huddart R, Loriot Y, et al. ESMO Clinical Practice Guideline interim update on first-line therapy in advanced urothelial carcinoma. Annals of Oncology. 2024 Jun;35(6):485-90.
14. Powles T, Valderrama BP, Gupta S, Bedke J, Kikuchi E, Hoffman-Censits J, et al. Enfortumab Vedotin and Pembrolizumab in Untreated Advanced Urothelial Cancer. New England Journal of Medicine. 2024 Mar 7;390(10):875-88.
15. van der Heijden MS, Sonpavde G, Powles T, Necchi A, Burotto M, Schenker M, et al. Nivolumab plus Gemcitabine-Cisplatin in Advanced Urothelial Carcinoma. New England Journal of Medicine. 2023 Nov 9;389(19):1778-89.
16. Balar A V, Castellano D, O'Donnell PH, Grivas P, Vuky J, Powles T, et al. First-line pembrolizumab in cisplatin-ineligible patients with locally advanced and unresectable or metastatic urothelial cancer (KEYNOTE-052): a multicentre, single-arm, phase 2 study. Lancet Oncol [Internet]. 2017 Nov [cited 2019 Oct 2];18(11):1483-92. Available from: https://linkinghub.elsevier.com/retrieve/pii/51470204517306162
17. Balar AV, Castellano DE, Grivas P, Vaughn DJ, Powles T, Vuky J, et al. Efficacy and safety of pembrolizumab in metastatic urothelial carcinoma: results from KEYNOTE-045 and KEYNOTE-052 after up to 5 years of follow-up. Annals of Oncology. 2023 Mar;34(3):289-99.
18. Powles T, Durán I, van der Heijden MS, Loriot Y, Vogelzang NJ, De Giorgi U, et al. Atezolizumab versus chemotherapy in patients with platinum-treated locally advanced or metastatic urothelial carcinoma (IMvigor211): a multicentre, open-label, phase 3 randomised controlled trial. The Lancet [Internet]. 2018 Feb 24 [cited 2021 Feb 11];391(10122):748-57. Available from: https://pubmed.ncbi.nlm.nih.gov/29268948/
19. Powles T, Kockx M, Rodriguez-Vida A, Duran I, Crabb SJ, Van Der Heijden MS, et al. Clinical efficacy and biomarker analysis of neoadjuvant atezolizumab in operable urothelial carcinoma in the ABACUS trial. Nat Med [Internet]. 2019 Nov 1 [cited 2021 Jun 25];25(11):1706-14. Available from: https://pubmed.ncbi.nlm.nih.gov/31686036/
20. Necchi A, Anichini A, Raggi D, Briganti A, Massa S, Lucianò R, et al. Pembrolizumab as Neoadjuvant Therapy Before Radical Cystectomy in Patients With Muscle-Invasive Urothelial Bladder Carcinoma (PURE-01): An Open-Label, Single-Arm, Phase II Study. J Clin Oncol [Internet]. 2018 Oct 20 [cited 2019 Oct 2];36(34):JC01801148. Available from: http://ascopubs.org/doi/10.1200/JCO.18.01148
21. Samstein RM, Lee CH, Shoushtari AN, Hellmann MD, Shen R, Janjigian YY, et al. Tumor mutational load predicts survival after immunotherapy across multiple cancer types. Nat Genet [Internet]. 2019 Feb 1 [cited 2021 Dec 19];51(2):202-6. Available from: https://pubmed.ncbi.nlm.nih.gov/30643254/
22. Rui X, Gu TT, Pan HF, Zhang HZ. Evaluation of PD-L1 biomarker for immune checkpoint inhibitor (PD-1/PD-L1 inhibitors) treatments for urothelial carcinoma patients: A meta-analysis. Int Immunopharmacol. 2019 Feb;67:378-85.
23. Kilgour E, Rothwell DG, Brady G, Dive C. Liquid Biopsy-Based Biomarkers of Treatment Response and Resistance. Cancer Cell. 2020 Apr 13;37(4):485-95.
24. Powles T, Chang YH, Yamamoto Y, Munoz J, Reyes-Cosmelli F, Peer A, et al. Pembrolizumab for advanced urothelial carcinoma: exploratory ctDNA biomarker analyses of the KEYNOTE-361 phase 3 trial. Nat Med. 2024 Jun 1;
25. Tolmeijer SH, van Wilpe S, Geerlings MJ, von Rhein D, Smilde TJ, Kloots lSH, et al. Early Ontreatment Circulating Tumor DNA Measurements and Response to immune Checkpoint Inhibitors in Advanced Urothelial Cancer. Eur Urol Oncol [Internet]. 2023 Sep [cited 2023 Oct 1]; Available from: https://pubmed.ncbi.nlm.nih.gov/37673768/
26. Hendrickx W, Simeone I, Anjum S, Mokrab Y, Bertucci F, Finetti P, et al. Identification of genetic determinants of breast cancer immune phenotypes by integrative genome-scale analysis. Oncoimmunology [Internet]. 2017 Feb 1 [cited 2024 Feb 29];6(2). Available from: https://pubmed.ncbi.nlm.nih.gov/28344865/
27. Seitz RS, Hurwitz ME, Nielsen TJ, Bailey DB, Varga MG, Ring BZ, et al. Translation of the 27-gene immuno-oncology test (IO score) to predict outcomes in immune checkpoint inhibitor treated metastatic urothelial cancer patients. J Transl Med [Internet]. 2022 Dec 1 [cited 2023 Sep 25];20(1). Available from: https://pubmed.ncbi.nlm.nih.gov/35974414/
28. Reijers ILM, Rao D, Versluis JM, Menzies AM, Dimitriadis P, Wouters MW, et al. IFN-γ signature enables selection of neoadjuvant treatment in patients with stage III melanoma. Journal of Experimental Medicine [Internet]. 2023 May 1 [cited 2023 Oct 2];220(5). Available from: https://doi.org/10.1084/jem.20221952
29. Schwartz LH, Litière S, de Vries E, Ford R, Gwyther S, Mandrekar S, et al. RECIST 1.1-Update and clarification: From the RECIST committee. Eur J Cancer [Internet]. 2016 Jul 1 [cited 2020 Apr 19];62:132-7. Available from: https://linkinghub.elsevier.com/retrieve/pii/S0959804916320433
30. Annala M, Vandekerkhove G, Khalaf D, Taavitsainen S, Beja K, Warner EW, et al. Circulating Tumor DNA Genomics Correlate with Resistance to Abiraterone and Enzalutamide in Prostate Cancer. Cancer Discov. 2018 Apr 1;8(4):444-57.
31. Powles T, Chang YH, Yamamoto Y, Munoz J, Reyes-Cosmelli F, Peer A, et al. Pembrolizumab for advanced urothelial carcinoma: exploratory ctDNA biomarker analyses of the KEYNOTE-361 phase 3 trial. Nat Med. 2024 Jun 1;
32. van Wilpe S, Wosika V, Ciarloni L, Ehrensberger SH, Jeitziner R, Angelino P, et al. Whole Blood Transcriptome Profiling Identifies DNA Replication and Cell Cycle Regulation as Early Marker of Response to Anti-PD-1 in Patients with Urothelial Cancer. Cancers (Basel) [Internet]. 2021 Sep 1 [cited 2021 Dec 16];13(18). Available from: https://pubmed.ncbi.nlm.nih.gov/34572887/
33. Herbst RS, Soria JC, Kowanetz M, Fine GD, Hamid O, Gordon MS, et al. Predictive correlates of response to the anti-PD-L1 antibody MPDL3280A in cancer patients. Nature. 2014 Nov 27;515(7528):563-7.
34. Strickler JH, Hanks BA, Khasraw M. Tumor Mutational Burden as a Predictor of Immunotherapy Response: Is More Always Better? Clinical Cancer Research. 2021 Mar 1;27(5):1236-41.
35. Fairfax BP, Taylor CA, Watson RA, Nassiri I, Danielli S, Fang H, et al. Peripheral CD8+ T cell characteristics associated with durable responses to immune checkpoint blockade in patients with metastatic melanoma. Nat Med [Internet]. 2020 Feb 1 [cited 2021 Dec 18];26(2):193. Available from: /pmc/articles/PMC7611047/
36. Luoma AM, Suo S, Wang Y, Gunasti L, Porter CBM, Nabilsi N, et al. Tissue-resident memory and circulating T cells are early responders to pre-surgical cancer immunotherapy. Cell. 2022 Aug; 185(16):2918-2935.e29.
37. Argelaguet R, Velten B, Arnol D, Dietrich S, Zenz T, Marioni JC, et al. Multi-Omics Factor Analysis-a framework for unsupervised integration of multi-omics data sets. Mol Syst Biol. 2018 Jun 20;14(6).
38. Sammut SJ, Crispin-Ortuzar M, Chin SF, Provenzano E, Bardwell HA, Ma W, et al. Multi-omic machine learning predictor of breast cancer therapy response. Nature. 2022 Jan 27;601(7894):623-9.

## Claims

1. A method for predicting or determining if a patient having a cancer will respond, or is responsive, to a treatment based on immune checkpoint blockade therapy or treatment (ICBT), said method comprising determining at one (t1) and/or more time points (t2, t3, ...) in one or more biological samples obtained from said patient having a cancer, the level of transcription and/or expression and/or activity of a gene panel of at least 5 genes, at least 7 genes, at least 10 genes selected from **Table 1**, and wherein differential transcription and/or expression and/or activity level of the gene panel relative to the level of corresponding transcription and/or expression and/or activity level of the gene panel determined previously, is predictive of the patient's response to said treatment.

2. The method of claim 1, wherein the gene panel comprises, or consists of, RAD51, SCARF1, SMPD3, CLC, CD86, PDXK, PTTG1, TOX, CEBPA and ENSG00000279447.

3. The method of any one of claims 1 to 2, wherein the differential transcription and/or expression and/or activity level of the gene panel is expressed as a gene expression metric.

4. The method of claim 3, wherein the gene expression metric is inputted into an algorithm that was trained using gene expression metric from patients having cancer, preferably the same cancer, and treated with a treatment based on ICBT and for which response status (responder, clinical benefit (CB)) or non-responder (non-clinical benefit (N-CB)) is known and wherein the trained algorithm further outputs a probability score.

5. The method of claim 4, wherein
- a probability score above a determined cutoff value indicates that the patient is determined as responsive, or will respond, to a treatment based on immune checkpoint blockade therapy or treatment (ICBT), or
- a probability score below a determined cutoff value indicates that the patient is determined as non responsive, or will not respond, to a treatment based on immune checkpoint blockade therapy or treatment (ICBT).

6. The method of any one of the preceding claims, wherein the biological samples are selected from the group comprising whole blood, serum, plasma, semen, saliva, tears, urine, fecal material, sweat, buccal smears, skin, tumor tissue, cancer cells, or a combination of two or more of thereof.

7. The method of any one of the preceding claims, wherein the cancer is selected from the group comprising urothelial cancer, urinary bladder cancer (e.g. muscle-invasive bladder cancer (MIBC)), lung cancer, breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, hepatic cancer, brain cancer and skin cancer (e.g. melanoma), or a combination of two or more thereof.

8. The method of any one of the preceding claims, wherein the treatment based on ICBT is selected from the group comprising a PD-1 inhibitor, a PD-L1 inhibitor, a TIM-3 inhibitor, a TIGIT inhibitor and a CTLA-4 inhibitor, or a combination of two or more thereof.

9. The method of any one of the preceding claims, further comprising determining at one (11) or more time points (t2, t3, ...) in one or more biological samples obtained from said patient having a cancer, the level and/or amount of circulating tumor DNA (ctDNA).

10. The method of any one of the preceding claims, wherein
- time point (t1) corresponds to a time point before starting a treatment based on ICBT or
- time point (t1) corresponds to a time point after starting a treatment based on ICBT.

11. The method of any one of the preceding claims, wherein timepoints t2, t3, .. correspond to one or more time points after 11, preferably after starting a treatment based on ICBT.

12. The method of claims 10 or 11, wherein
- times points (t1, t2, t3, *etc*...) correspond to the same time points as those used for determining the level of transcription and/or expression and/or activity of the genes selected from **Table 1,** or
- times points (t1, t2, t3, *etc*...) do not correspond to the same time points as those used for determining the level of transcription and/or expression and/or activity of the genes selected from **Table 1.**

13. The method of any one of claims 9 to 12, wherein differential level and/or amount of ctDNAs, in the biological sample, determined at one (11) or more time points (t2, t3, ...) after starting a treatment based on ICBT relative to corresponding level and/or amount of ctDNAs determined previously, is predictive of the patient's response to said treatment.

14. The method of claim 13, wherein the differential level and/or amount of is expressed as a metric.

15. The method of any one of the preceding claims, wherein the level of corresponding transcription and/or expression and/or activity level of the gene panel and/or level and/or amount of ctDNAs determined previously correspond to a control sample, a reference sample, a group of reference samples, a reference value, a reference score or a value determined previously at an earlier time point, preferably before starting the treatment for the same patient (such as e.g. at 11).

16. The method of anyone of claims 4 to 15, wherein
i) if the probability score is high confidence, the probability score is considered to determine whether the patient is responsive (responder, having clinical benefit (CB)) or not responsive (non-responder, patient not having clinical benefit (N-CB)) to said treatment,
ii) if the probability score is low confidence, then the differential level and/or amount of ctDNAs is also considered to determine whether the patient is responsive (responder, having clinical benefit (CB)) or not responsive (non-responder, patient not having clinical benefit (N-CB)) to said treatment, or
iii) if the probability score and the ctDNA metric are low confidence, then the probability score or the differential level and/or amount of ctDNAs is considered to determine whether the patient is responsive (responder, having clinical benefit (CB)) or not responsive (non-responder, patient not having clinical benefit (N-CB)) to said treatment.

17. The method of any one of the preceding claims, wherein,
i) if the patient having a cancer is determined as responsive (responder, CB) to said treatment based on ICBT, the treatment is continued, or
ii) if the patient having a cancer is determined as not responsive (non-responder, N-CB) to said treatment based on ICBT, the method further comprises a step of adapting the treatment.

18. The method of any one of the preceding claims, wherein
i) the level of transcription and/or expression and/or activity of the gene panel is detected in one or more biological sample about 1 to about 16 weeks, about 2 to about 14 weeks, about 2 to about 12 weeks, about 2 to about 10 weeks after the treatment based on ICBT has started,
ii) the level and/or amount of ctDNAs is determined in one or more biological sample between about 1 to about 16 weeks, about 2 to about 14 weeks, about 2 to about 12 weeks, about 2 to about 10 weeks, after the treatment based on ICBT has started,
iii) the level of transcription and/or expression and/or activity of the gene panel is detected before the treatment based on ICBT has started, preferably between about 1 to about 5 hours, about 1 to about 16 weeks, about 2 to about 14 weeks, about 2 to about 12 weeks, about 2 to about 10 weeks, and/or
iv) the level and/or amount of ctDNAs is determined in one or more biological sample before the treatment based on ICBT has started, preferably between about 1 to about 5 hours, about 1 to about 16 weeks, about 2 to about 14 weeks, about 2 to about 12 weeks, about 2 to about 10 weeks.

19. A method for predicting and/or determining if a patient having a cancer will respond, or is responsive, to a treatment based on immune checkpoint blockade therapy or treatment (ICBT), said method comprising,
i) determining at one (t1) or more time points (t2, t3, ...) the level of transcription and/or expression and/or activity of at least one gene in one or more biological samples obtained from said patient having a cancer,
ii) determining a differential transcription and/or expression and/or activity level of the at least one gene by comparing the transcription and/or expression and/or activity level at one (11) or more time points (t2, t3, ...) versus the transcription and/or expression and/or activity level determined previously,
iii) expressing said differential transcription and/or expression and/or activity level as a gene expression metric,
iv) classifying the samples according to the gene expression metric using an algorithm trained on expression data obtained from samples labelled as responder(s) (CB) and non-responder(s) (N-CB) and, optionally, selecting a gene panel comprising at least one gene responding to treatment based on immune checkpoint blockade therapy or treatment (ICBT),
v) determining at one (11) or more time points (t2, t3, ...) in one or more biological samples obtained from said patient having a cancer, the level and/or amount of circulating tumor DNA (ctDNA),
vi) determining a differential level and/or amount of ctDNAs by comparing the level and/or amount at one (11) or more time points (t2, t3, ...) versus the level and/or amount determined previously, and
wherein differential transcription and/or expression and/or activity level of the genes, or the gene panel, relative to the level of corresponding transcription and/or expression and/or activity level of the genes or gene panel determined previously, and
wherein differential level and/or amount of ctDNAs, in the biological sample, determined at one or more time points (t2, t3, ...) after starting a treatment based on ICBT relative to corresponding level and/or amount of ctDNAs determined previously,
are predictive of the patient's response to said treatment.
